# EUROPEAN PATENT SPECIFICATION

(11) **EP 3 240 799 B1**
(45) Date of publication and mention of the grant of the patent: **31.03.2021**
(21) Application number: 15876063.7
(22) Date of filing: 27.12.2015
(51) Int. Cl.: C07K 14/575, A61K 38/00, A61P 3/00, A61P 7/06, A61P 43/00

(54) **S-ALKYLATED HEPCIDIN PEPTIDES AND METHODS OF MAKING AND USING THEREOF**
S-ALKYLIERTE HEPCIDIN-PEPTIDE UND VERFAHREN ZUR HERSTELLUNG UND VERWENDUNG DAVON
PEPTIDES HEPCIDINE S-ALKYLÉS ET LEURS PROCÉDÉS DE PRÉPARATION ET D'UTILISATION

(30) Priority: 29.12.2014 US 201462097429 P
(43) Date of publication of application: 08.11.2017
(73) Proprietor: The Regents of the University of California, Oakland, CA 94607-5200 (US)
(72) Inventor: RUCHALA, Piotr, Los Angeles, CA 90095-7191 (US); GANZ, Tomas, Los Angeles, CA 90095-7191 (US); NEMETH, Elizabeta, Los Angeles, CA 90095-7191 (US)
(74) Representative: HGF
(86) International application number: PCT/US2015/067545
(87) International publication number: WO 2016/109363

(56) References cited:
- WO-A1-2013/086143
- WO-A2-2010/065815
- US-A1- 2014 336 110
- NEMETH ELIZABETA ET AL: "The N-terminus of hepcidin is essential for its interaction with ferroportin: structure-function study", [Online] 1 January 2006 (2006-01-01), BLOOD, AMERICAN SOCIETY OF HEMATOLOGY, US, PAGE(S) 328 - 333, XP002519931, ISSN: 0006-4971 Retrieved from the Internet: URL:10.1182/BLOOD-2005-05-2049> [retrieved on 2005-09-01] * page 329, right-hand column, paragraph 4 - page 331, right-hand column, paragraph 1 *
- KRISTINE CHUA ET AL: "Small cyclic agonists of iron regulatory hormone hepcidin", BIOORGANIC & MEDICINAL CHEMISTRY LETTERS, vol. 25, no. 21, 12 March 2015 (2015-03-12), pages 4961-4969, XP055356359, AMSTERDAM, NL ISSN: 0960-894X, DOI: 10.1016/j.bmcl.2015.03.012
- FUNG ET AL.: 'Thiol-derivatized minihepcidins retain biological activity' BIOORG MED CHEM LETT. vol. 25, no. 4, 15 February 2015, pages 763 - 766, XP055460384
- THUMFORT ET AL.: 'S-Ethenyl Cysteine; An Amino Acid From Olax phyllanthi' PHYTOCHEMISTRY vol. 34, no. 3, 01 October 1993, pages 657 - 659, XP026660946
- PREZA ET AL.: 'MINIHEPCIDINS ARE RATIONALLY DESIGNED SMALL PEPTIDES THAT MIMIC HEPCIDIN ACTIVITY IN MICE AND MAY BE USEFUL FOR THE TREATMENT OF IRON OVERLOAD' THE JOURNAL OF CLINICAL INVESTIGATION vol. 121, no. 12, 01 December 2011, pages 4880 - 4889, XP055024189
- TRIOLA ET AL.: 'Racemization-Free Synthesis of S-Alkylated Cysteines via Thiol-ene Reaction' J. ORG. CHEM. vol. 73, no. 9, 01 April 2008, pages 3646 - 3649, XP055014212

### SEQUENCE LISTING

<110> The Regents of the University of California
<120> S-ALKYLATED HEPCIDIN PEPTIDES AND METHODS OF MAKING AND USING THEREOF
<130> 034044.155WO1
<150> US 62/097,429
   <151> 2014-12-29
<160> 101
<170> PatentIn version 3.5
<210> 1
   <211> 25
   <212> PRT
   <213> Homo sapiens
<400> 1
   Asp Thr His Phe Pro Ile Cys Ile Phe Cys Cys Gly Cys Cys His Arg
   1               5                   10                  15
   Ser Lys Cys Gly Met Cys Cys Lys Thr
               20                  25
<210> 2
   <211> 10
   <212> PRT
   <213> Artificial Sequence
<220>
   <223> Based on Homo sapiens sequence
<400> 2
   Asp Thr His Phe Pro Ile Cys Ile Phe Cys
   1 5 10
<210> 3
   <211> 4
   <212> PRT
   <213> Artificial Sequence
<220>
   <223> Based on Homo sapiens sequence
<400> 3
   Phe Pro Ile Cys
   1
<210> 4
   <211> 5
   <212> PRT
   <213> Artificial Sequence
<220>
   <223> Based on Homo sapiens sequence
<400> 4
   His Phe Pro Ile Cys
   1 5
<210> 5
   <211> 6
   <212> PRT
   <213> Artificial Sequence
<220>
   <223> Based on Homo sapiens sequence
<400> 5
   His Phe Pro Ile Cys Ile
   1 5
<210> 6
   <211> 7
   <212> PRT
   <213> Artificial Sequence
<220>
   <223> Based on Homo sapiens sequence
<400> 6
   His Phe Pro Ile Cys Ile Phe
   1 5
<210> 7
   <211> 7
   <212> PRT
   <213> Artificial Sequence
<220>
   <223> Based on Homo sapiens sequence
<400> 7
   Asp Thr His Phe Pro Ile Cys
   1               5
<210> 8
   <211> 8
   <212> PRT
   <213> Artificial Sequence
<220>
   <223> Based on Homo sapiens sequence
<400> 8
   Asp Thr His Phe Pro Ile Cys Ile
   1               5
<210> 9
   <211> 9
   <212> PRT
   <213> Artificial Sequence
<220>
   <223> Based on Homo sapiens sequence
<400> 9
   Asp Thr His Phe Pro Ile Cys Ile Phe
   1               5
<210> 10
   <211> 10
   <212> PRT
   <213> Artificial Sequence
<220>
   <223> Based on Homo sapiens sequence
<400> 10
   Asp Thr His Phe Pro Ile Ala Ile Phe Cys
   1               5                   10
<210> 11
   <211> 9
   <212> PRT
   <213> Artificial Sequence
<220>
   <223> Based on Homo sapiens sequence
<400> 11
   Asp Thr His Ala Pro Ile Cys Ile Phe
   1 5
<210> 12
   <211> 9
   <212> PRT
   <213> Artificial Sequence
<220>
   <223> Based on Homo sapiens sequence
<220>
   <221> misc_feature
   <222> (7)..(7)
   <223> Xaa is S-t-butylthio-L-cysteine
<400> 12
   Asp Thr His Ala Pro Ile Xaa Ile Phe
   1 5
<210> 13
   <211> 9
   <212> PRT
   <213> Artificial Sequence
<220>
   <223> Based on Homo sapiens sequence
<220>
   <221> misc_feature
   <222> (7)..(7)
   <223> Xaa is t-butylthio-L-cysteine
<400> 13
   Asp Thr His Ala Pro Ile Xaa Ile Phe
   1 5
<210> 14
   <211> 9
   <212> PRT
   <213> Artificial Sequence
<220>
   <223> Based on Homo sapiens sequence
<400> 14
   Asp Thr His Phe Pro Ile Ala Ile Phe
   1 5
<210> 15
   <211> 9
   <212> PRT
   <213> Artificial Sequence
<220>
   <223> Based on Homo sapiens sequence
<400> 15
   Asp Thr His Phe Pro Ile Ser Ile Phe
   1               5
<210> 16
   <211> 9
   <212> PRT
   <213> Artificial Sequence
<220>
   <223> Based on Homo sapiens sequence
<220>
   <221> misc_feature
   <222> (7)..(7)
   <223> D-amino acid
<400> 16
   Asp Thr His Phe Pro Ile Cys Ile Phe
   1               5
<210> 17
   <211> 9
   <212> PRT
   <213> Artificial Sequence
<220>
   <223> Based on Homo sapiens sequence
<220>
   <221> misc_feature
   <222> (7)..(7)
   <223> Xaa is L-homocysteine
<400> 17
   Asp Thr His Ala Pro Ile Xaa Ile Phe
   1               5
<210> 18
   <211> 9
   <212> PRT
   <213> Artificial Sequence
<220>
   <223> Based on Homo sapiens sequence
<220>
   <221> misc_feature
   <222> (7)..(7)
   <223> Xaa is L-penicillamine
<400> 18
   Asp Thr His Ala Pro Ile Xaa Ile Phe
   1               5
<210> 19
   <211> 9
   <212> PRT
   <213> Artificial Sequence
<220>
   <223> Based on Homo sapiens sequence
<220>
   <221> misc_feature
   <222> (7)..(7)
   <223> Xaa is D-penicillamine
<400> 19
   Asp Thr His Phe Pro Ile Xaa Ile Phe
   1               5
<210> 20
   <211> 9
   <212> PRT
   <213> Artificial Sequence
<220>
   <223> Based on Homo sapiens sequence
<220>
   <221> misc_feature
   <222> (7)..(7)
   <223> Xaa is N-(bromoacetyl)-L-2,3-diaminopropionic acid
<400> 20
   Asp Thr His Ala Pro Ile Xaa Ile Phe
   1               5
<210> 21
   <211> 9
   <212> PRT
   <213> Artificial Sequence
<220>
   <223> Based on Homo sapiens sequence
<220>
   <221> misc_feature
   <222> (2)..(2)
   <223> Xaa is L-tert-leucine
<220>
   <221> misc_feature
   <222> (4)..(4)
   <223> Xaa is L-phenylglycine
<220>
   <221> misc_feature
   <222> (5)..(5)
   <223> Xaa is octahydroindole-2-carboxylic acid
<220>
   <221> misc_feature
   <222> (6)..(6)
   <223> Xaa is L-alpha-cyclohexylglycine
<220>
   <221> misc_feature
   <222> (8)..(8)
   <223> Xaa is L-alpha-cyclohexylglycine
<400> 21
   Asp Xaa His Xaa Xaa Xaa Cys Xaa Phe
   1               5
<210> 22
   <211> 9
   <212> PRT
   <213> Artificial Sequence
<220>
   <223> Based on Homo sapiens sequence
<220>
   <221> misc_feature
   <222> (2)..(2)
   <223> Xaa is L-tert-leucine
<220>
   <221> misc_feature
   <222> (5)..(5)
   <223> Xaa is octahydroindole-2-carboxylic acid
<220>
   <221> misc_feature
   <222> (6)..(6)
   <223> Xaa is L-alpha-cyclohexylglycine
<220>
   <221> misc_feature
   <222> (8)..(8)
   <223> Xaa is L-alpha-cyclohexylglycine
<400> 22
   Asp Xaa His Pro Xaa Xaa Cys Xaa Phe
   1               5
<210> 23
   <211> 9
   <212> PRT
   <213> Artificial Sequence
<220>
   <223> Based on Homo sapiens sequence
<220>
   <221> misc_feature
   <222> (1)..(9)
   <223> D-amino aciids
<400> 23
   Phe Ile Cys Ile Pro Phe His Thr Asp
   1               5
<210> 24
   <211> 7
   <212> PRT
   <213> Artificial Sequence
<220>
   <223> Based on Homo sapiens sequence
<220>
   <221> misc_feature
   <222> (1)..(7)
   <223> D-amino acids
<400> 24
   Phe Ile Cys Ile Pro Phe His
   1               5
<210> 25
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (1)..(1)
   <223> Having chenodeoxycholate-(D)Asp-(PEG11)- bound thereto
<400> 25
   Phe Ile Cys Ile Pro Phe His Thr Asp
   1               5
<210> 26
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (1)..(1)
   <223> Having ursodeoxycholate-(D)Asp-(PEG11)- bound thereto
<400> 26
   Phe Ile Cys Ile Pro Phe His Thr Asp
   1               5
<210> 27
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (9)..(9)
   <223> Having -(PEG11)-GYIPEAPRDGQAYVRKDGEWVLLSTFL bound thereto
<400> 27
   Phe Ile Cys Ile Pro Phe His Thr Asp
   1               5
<210> 28
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (9)..(9)
   <223> Having -(PEG11)-(GPHyp)10 bound thereto
<400> 28
   Phe Ile Cys Ile Pro Phe His Thr Asp
   1               5
<210> 29
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (1)..(1)
   <223> Having palmitoyl-(PEG11)- bound thereto
<400> 29
   Phe Ile Cys Ile Pro Phe His Thr Asp
   1               5
<210> 30
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (1)..(1)
   <223> Having (Palmitoyl)2-Dap-PEG11- bound thereto
<400> 30
   Phe Ile Cys Ile Pro Phe His Thr Asp
   1               5
<210> 31
   <211> 9
   <212> PRT
   <213> Artificial Sequence
<220>
   <223> Based on Homo sapiens sequence
<220>
   <221> misc_feature
   <222> (4)..(4)
   <223> Xaa is beta-homophenylalanine
<400> 31
   Asp Thr His Xaa Pro Ile Cys Ile Phe
   1               5
<210> 32
   <211> 9
   <212> PRT
   <213> Artificial Sequence
<220>
   <223> Based on Homo sapiens sequence
<220>
   <221> misc_feature
   <222> (4)..(4)
   <223> Xaa is 3,3-diphenyl-L-alanine
<400> 32
   Asp Thr His Xaa Pro Ile Cys Ile Phe
   1               5
<210> 33
   <211> 9
   <212> PRT
   <213> Artificial Sequence
<220>
   <223> Based on Homo sapien sequence
<220>
   <221> misc_feature
   <222> (4)..(4)
   <223> Xaa is L-biphenylalanine
<400> 33
   Asp Thr His Xaa Pro Ile Cys Ile Phe
   1               5
<210> 34
   <211> 9
   <212> PRT
   <213> Artificial Sequence
<220>
   <223> Based on Homo sapiens sequence
<220>
   <221> misc_feature
   <222> (4)..(4)
   <223> Xaa is (1-naphthyl)-L-alanine
<400> 34
   Asp Thr His Xaa Pro Ile Cys Ile Phe
   1               5
<210> 35
   <211> 9
   <212> PRT
   <213> Artificial Sequence
<220>
   <223> Based on Homo sapiens sequence
<220>
   <221> misc_feature
   <222> (4)..(4)
   <223> Xaa is (S)-3-Amino-4,4-diphenylbutanoic acid
<400> 35
   Asp Thr His Xaa Pro Ile Cys Ile Phe
   1               5
<210> 36
   <211> 9
   <212> PRT
   <213> Artificial Sequence
<220>
   <223> Based on Homo sapiens sequence
<220>
   <221> misc_feature
   <222> (9)..(9)
   <223> Xaa is beta-homophenylalanine
<400> 36
   Asp Thr His Phe Pro Ile Cys Ile Xaa
   1               5
<210> 37
   <211> 9
   <212> PRT
   <213> Artificial Sequence
<220>
   <223> Based on Homo sapiens sequence
<220>
   <221> misc_feature
   <222> (9)..(9)
   <223> Xaa is 3,3-diphenyl-L-alanine
<400> 37
   Asp Thr His Phe Pro Ile Cys Ile Xaa
   1               5
<210> 38
   <211> 9
   <212> PRT
   <213> Artificial Sequence
<220>
   <223> Based on Homo sapiens sequence
<220>
   <221> misc_feature
   <222> (9)..(9)
   <223> Xaa is L-biphenylalanine
<400> 38
   Asp Thr His Phe Pro Ile Cys Ile Xaa
   1               5
<210> 39
   <211> 9
   <212> PRT
   <213> Artificial Sequence
<220>
   <223> Based on Homo sapiens sequence
<220>
   <221> misc_feature
   <222> (9)..(9)
   <223> Xaa is (1-naphthyl)-L-alanine
<400> 39
   Asp Thr His Phe Pro Ile Cys Ile Xaa
   1               5
<210> 40
   <211> 9
   <212> PRT
   <213> Artificial Sequence
<220>
   <223> Based on Homo sapiens sequence
<220>
   <221> misc_feature
   <222> (9)..(9)
   <223> Xaa is (S)-3-Amino-4,4-diphenylbutanoic acid
<400> 40
   Asp Thr His Phe Pro Ile Cys Ile Xaa
   1 5
<210> 41
   <211> 9
   <212> PRT
   <213> Artificial Sequence
<220>
   <223> Based on Homo sapiens sequence
<220>
   <221> misc_feature
   <222> (4)..(4)
   <223> Xaa is 3,3-diphenyl-L-alanine
<220>
   <221> misc_feature
   <222> (9)..(9)
   <223> Xaa is 3,3-diphenyl-L-alanine
<400> 41
   Asp Thr His Xaa Pro Ile Cys Ile Xaa
   1 5
<210> 42
   <211> 7
   <212> PRT
   <213> Artificial Sequence
<220>
   <223> Based on Homo sapiens sequence
<220>
   <221> misc_feature
   <222> (2)..(2)
   <223> Xaa is 3,3-diphenyl-L-alanine
<400> 42
   Asp Xaa Pro Ile Cys Ile Phe
   1 5
<210> 43
   <211> 7
   <212> PRT
   <213> Artificial Sequence
<220>
   <223> Based on Homo sapiens sequence
<220>
   <221> misc_feature
   <222> (2)..(2)
   <223> Xaa is 3,3-diphenyl-L-alanine
<220>
   <221> misc_feature
   <222> (7)..(7)
   <223> Xaa is 3,3-diphenyl-L-alanine
<400> 43
   Asp Xaa Pro Ile Cys Ile Xaa
   1               5
<210> 44
   <211> 9
   <212> PRT
   <213> Artificial Sequence
<220>
   <223> Based on Homo sapiens sequence
<220>
   <221> misc_feature
   <222> (4)..(4)
   <223> Xaa is 3,3-diphenyl-L-alanine
<220>
   <221> misc_feature
   <222> (6)..(6)
   <223> D-amino acid
<220>
   <221> misc_feature
   <222> (8)..(8)
   <223> D-amino acid
<220>
   <221> misc_feature
   <222> (9)..(9)
   <223> Xaa is 3,3-diphenyl-L-alanine
<400> 44
   Asp Thr His Xaa Pro Arg Cys Arg Xaa
   1               5
<210> 45
   <211> 9
   <212> PRT
   <213> Artificial Sequence
<220>
   <223> Based on Homo sapiens sequence
<220>
   <221> misc_feature
   <222> (4)..(4)
   <223> Xaa is 3,3-diphenyl-L-alanine
<220>
   <221> misc_feature
   <222> (5)..(5)
   <223> Xaa is octahydroindole-2-carboxylic acid
<400> 45
   Asp Thr His Xaa Xaa Ile Cys Ile Phe
   1               5
<210> 46
   <211> 9
   <212> PRT
   <213> Artificial Sequence
<220>
   <223> Based on Homo sapiens sequence
<220>
   <221> misc_feature
   <222> (4)..(4)
   <223> Xaa is 3,3-diphenyl-L-alanine
<220>
   <221> misc_feature
   <222> (5)..(5)
   <223> Xaa is octahydroindole-2-carboxylic acid
<220>
   <221> misc_feature
   <222> (9)..(9)
   <223> Xaa is 3,3-diphenyl-L-alanine
<400> 46
   Asp Thr His Xaa Xaa Ile Cys Ile Xaa
   1               5
<210> 47
   <211> 9
   <212> PRT
   <213> Artificial Sequence
<220>
   <223> Based on Homo sapiens sequence
<220>
   <221> misc_feature
   <222> (4)..(4)
   <223> Xaa is 3,3-diphenyl-L-alanine
<220>
   <221> misc_feature
   <222> (9)..(9)
   <223> Xaa is 3,3-diphenyl-L-alanine
<400> 47
   Asp Thr His Xaa Pro Cys Cys Cys Xaa
   1               5
<210> 48
   <211> 12
   <212> PRT
   <213> Artificial Sequence
<220>
   <223> Based on Homo sapiens sequence
<220>
   <221> misc_feature
   <222> (10)..(10)
   <223> D-amino acid
<400> 48
   Asp Thr His Phe Pro Ile Cys Ile Phe Pro Pro Lys
   1               5                   10
<210> 49
   <211> 12
   <212> PRT
   <213> Artificial Sequence
<220>
   <223> Based on Homo sapiens sequence
<220>
   <221> misc_feature
   <222> (10)..(10)
   <223> D-amino acid
<400> 49
   Asp Thr His Phe Pro Ile Cys Ile Phe Pro Pro Arg
   1               5                   10
<210> 50
   <211> 12
   <212> PRT
   <213> Artificial Sequence
<220>
   <223> Based on Homo sapiens sequence
<220>
   <221> misc_feature
   <222> (10)..(10)
   <223> Xaa is L-beta-homoproline
<400> 50
   Asp Thr His Phe Pro Ile Cys Ile Phe Xaa Pro Lys
   1               5                   10
<210> 51
   <211> 12
   <212> PRT
   <213> Artificial Sequence
<220>
   <223> Based on Homo sapiens sequence
<220>
   <221> misc_feature
   <222> (10)..(10)
   <223> Xaa is L-beta-homoproline
<400> 51
   Asp Thr His Phe Pro Ile Cys Ile Phe Xaa Pro Arg
   1               5                   10
<210> 52
   <211> 12
   <212> PRT
   <213> Artificial Sequence
<220>
   <223> Based on Homo sapiens sequence
<220>
   <221> misc_feature
   <222> (10)..(10)
   <223> D-amino acid
<220>
   <221> misc_feature
   <222> (11)..(11)
   <223> Xaa is L-beta-homoproline
<400> 52
   Asp Thr His Phe Pro Ile Cys Ile Phe Pro Xaa Lys
   1               5                   10
<210> 53
   <211> 12
   <212> PRT
   <213> Artificial Sequence
<220>
   <223> Based on Homo sapiens sequence
<220>
   <221> misc_feature
   <222> (10)..(10)
   <223> D-amino acid
<220>
   <221> misc_feature
   <222> (11)..(11)
   <223> Xaa is L-beta-homoproline
<400> 53
   Asp Thr His Phe Pro Ile Cys Ile Phe Pro Xaa Arg
   1               5                   10
<210> 54
   <211> 12
   <212> PRT
   <213> Artificial Sequence
<220>
   <223> Based on Homo sapiens sequence
<220>
   <221> misc_feature
   <222> (9)..(9)
   <223> Xaa is beta-homophenylalanine
<220>
   <221> misc_feature
   <222> (10)..(10)
   <223> D-amino acid
<400> 54
   Asp Thr His Phe Pro Ile Cys Ile Xaa Pro Pro Lys
   1               5                   10
<210> 55
   <211> 12
   <212> PRT
   <213> Artificial Sequence
<220>
   <223> Based on Homo sapiens sequence
<220>
   <221> misc_feature
   <222> (9)..(9)
   <223> Xaa is beta-homophenylalanine
<220>
   <221> misc_feature
   <222> (10)..(10)
   <223> L-amino acid
<220>
   <221> misc_feature
   <222> (10)..(10)
   <223> D-amino acid
<400> 55
   Asp Thr His Phe Pro Ile Cys Ile Xaa Pro Pro Arg
   1               5                   10
<210> 56
   <211> 12
   <212> PRT
   <213> Artificial Sequence
<220>
   <223> Based on Homo sapiens sequence
<220>
   <221> misc_feature
   <222> (9)..(9)
   <223> Xaa is beta-homophenylalanine
<220>
   <221> misc_feature
   <222> (10)..(10)
   <223> D-amino acid
<220>
   <221> misc_feature
   <222> (11)..(11)
   <223> Xaa is L-beta-homoproline
<400> 56
   Asp Thr His Phe Pro Ile Cys Ile Xaa Pro Xaa Lys
   1               5                   10
<210> 57
   <211> 12
   <212> PRT
   <213> Artificial Sequence
<220>
   <223> Based on Homo sapiens sequence
<220>
   <221> misc_feature
   <222> (9)..(9)
   <223> Xaa is beta-homophenylalanine
<220>
   <221> misc_feature
   <222> (10)..(10)
   <223> D-amino acid
<220>
   <221> misc_feature
   <222> (11)..(11)
   <223> Xaa is L-beta-homoproline
<400> 57
   Asp Thr His Phe Pro Ile Cys Ile Xaa Pro Xaa Arg
   1               5                   10
<210> 58
   <211> 9
   <212> PRT
   <213> Artificial Sequence
<220>
   <223> Based on Homo sapiens sequence
<220>
   <221> misc_feature
   <222> (2)..(2)
   <223> Xaa is isonipecotic acid
<220>
   <221> misc_feature
   <222> (3)..(3)
   <223> Xaa is 3,3-diphenyl-D-alanine
<220>
   <221> misc_feature
   <222> (4)..(4)
   <223> Xaa is (aminomethyl)cyclohexane carboxylic acid
<220>
   <221> misc_feature
   <222> (6)..(6)
   <223> Xaa is (aminomethyl)cyclohexane carboxylic acid
<220>
   <221> misc_feature
   <222> (7)..(7)
   <223> Xaa is isonipecotic acid
<220>
   <221> misc_feature
   <222> (8)..(8)
   <223> Xaa is 3,3-diphenyl-L-alanine
<220>
   <221> misc_feature
   <222> (9)..(9)
   <223> Xaa is cysteamide, oxidized
<400> 58
   Cys Xaa Xaa Xaa Arg Xaa Xaa Xaa Xaa
   1               5
<210> 59
   <211> 9
   <212> PRT
   <213> Artificial Sequence
<220>
   <223> Based on Homo sapiens sequence
<220>
   <221> misc_feature
   <222> (3)..(3)
   <223> Xaa is 3,3-diphenyl-D-alanine
<220>
   <221> misc_feature
   <222> (4)..(4)
   <223> Xaa can be any naturally occurring amino acid
<220>
   <221> misc_feature
   <222> (6)..(6)
   <223> Xaa can be any naturally occurring amino acid
<220>
   <221> misc_feature
   <222> (7)..(7)
   <223> Xaa is isonipecotic acid
<220>
   <221> misc_feature
   <222> (8)..(8)
   <223> Xaa is 3,3-diphenyl-L-alanine
<220>
   <221> misc_feature
   <222> (9)..(9)
   <223> Xaa is cysteamide, oxidized
<400> 59
   Cys Pro Xaa Xaa Arg Xaa Xaa Xaa Xaa
   1               5
<210> 60
   <211> 9
   <212> PRT
   <213> Artificial Sequence
<220>
   <223> Based on Homo sapiens sequence
<220>
   <221> misc_feature
   <222> (2)..(2)
   <223> D-amino acid
<220>
   <221> misc_feature
   <222> (3)..(3)
   <223> Xaa is 3,3-diphenyl-D-alanine
<220>
   <221> misc_feature
   <222> (4)..(4)
   <223> Xaa is (aminomethyl)cyclohexane carboxylic acid
<220>
   <221> misc_feature
   <222> (6)..(6)
   <223> Xaa is (aminomethyl)cyclohexane carboxylic acid
<220>
   <221> misc_feature
   <222> (7)..(7)
   <223> Xaa is isonipecotic acid
<220>
   <221> misc_feature
   <222> (8)..(8)
   <223> Xaa is 3,3-diphenyl-L-alanine
<220>
   <221> misc_feature
   <222> (9)..(9)
   <223> Xaa is cysteamide, oxidized
<400> 60
   Cys Pro Xaa Xaa Arg Xaa Xaa Xaa Xaa
   1               5
<210> 61
   <211> 9
   <212> PRT
   <213> Artificial Sequence
<220>
   <223> Based on Homo sapiens sequence
<220>
   <221> misc_feature
   <222> (3)..(3)
   <223> Xaa is 3,3-diphenyl-D-alanine
<220>
   <221> misc_feature
   <222> (4)..(4)
   <223> Xaa is (aminomethyl)cyclohexane carboxylic acid
<220>
   <221> misc_feature
   <222> (6)..(6)
   <223> Xaa is (aminomethyl)cyclohexane carboxylic acid
<220>
   <221> misc_feature
   <222> (7)..(7)
   <223> Xaa is isonipecotic acid
<220>
   <221> misc_feature
   <222> (8)..(8)
   <223> Xaa is 3,3-diphenyl-L-alanine
<220>
   <221> misc_feature
   <222> (9)..(9)
   <223> Xaa is cysteamide, oxidized
<400> 61
   Cys Gly Xaa Xaa Arg Xaa Xaa Xaa Xaa
   1               5
<210> 62
   <211> 27
   <212> PRT
   <213> Artificial Sequence
<220>
   <223> Based on Homo sapiens sequence
<220>
   <221> misc_feature
   <222> (1)..(1)
   <223> Having -(PEG11)- bound thereto
<400> 62
   Gly Tyr Ile Pro Glu Ala Pro Arg Asp Gly Gln Ala Tyr Val Arg Lys
   1               5                   10                  15
   Asp Gly Glu Trp Val Leu Leu Ser Thr Phe Leu
               20                  25
<210> 63
   <211> 9
   <212> PRT
   <213> Artificial Sequence
<220>
   <223> Based on Homo sapiens sequence
<220>
   <221> misc_feature
   <222> (4)..(4)
   <223> Xaa is 3,3-diphenyl-L-alanine
<220>
   <221> misc_feature
   <222> (9)..(9)
   <223> Xaa is 3,3-diphenyl-L-alanine
<400> 63
   Asp Thr His Xaa Pro Arg Cys Arg Xaa
   1               5
<210> 64
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (4)..(4)
   <223> Xaa is 3,3-diphenyl-L-alanine
<220>
   <221> misc_feature
   <222> (9)..(9)
   <223> Having palmitoyl-(PEG11)- bound thereto
<400> 64
   Asp Thr His Xaa Pro Ile Cys Ile Phe
   1 5
<210> 65
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (4)..(4)
   <223> Xaa is 3,3-diphenyl-L-alanine
<220>
   <221> misc_feature
   <222> (9)..(9)
   <223> Having palmitoyl-(PEG11)- bound thereto
<220>
   <221> misc_feature
   <222> (9)..(9)
   <223> Xaa is 3,3-diphenyl-L-alanine
<400> 65
   Asp Thr His Xaa Pro Ile Cys Ile Xaa
   1 5
<210> 66
   <211> 7
   <212> PRT
   <213> Artificial Sequence
<220>
   <223> Based on Homo sapiens sequence
<220>
   <221> misc_feature
   <222> (1)..(7)
   <223> D-amino acids
<220>
   <221> misc_feature
   <222> (2)..(2)
   <223> Xaa is 3,3-diphenyl-L-alanine
<220>
   <221> misc_feature
   <222> (7)..(7)
   <223> Having palmitoyl-(PEG11)- bound thereto
<400> 66
   His Xaa Pro Ile Cys Ile Phe
   1               5
<210> 67
   <211> 7
   <212> PRT
   <213> Artificial Sequence
<220>
   <223> Based on Homo sapiens sequence
<220>
   <221> misc_feature
   <222> (1)..(7)
   <223> D-amino acids
<220>
   <221> misc_feature
   <222> (2)..(2)
   <223> Xaa is 3,3-diphenyl-L-alanine
<220>
   <221> misc_feature
   <222> (7)..(7)
   <223> Having palmitoyl-(PEG11)- bound thereto
<220>
   <221> misc_feature
   <222> (7)..(7)
   <223> Xaa is 3,3-diphenyl-L-alanine
<400> 67
   His Xaa Pro Ile Cys Ile Xaa
   1               5
<210> 68
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (4)..(4)
   <223> Xaa is 3,3-diphenyl-L-alanine
<220>
   <221> misc_feature
   <222> (9)..(9)
   <223> Having palmitoyl-(PEG11)- bound thereto
<400> 68
   Asp Thr His Xaa Pro Val Cys Val Phe
   1 5
<210> 69
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (4)..(4)
   <223> Xaa is 3,3-diphenyl-L-alanine
<220>
   <221> misc_feature
   <222> (9)..(9)
   <223> Having palmitoyl-(PEG11)- bound thereto
<400> 69
   Asp Thr His Xaa Pro Leu Cys Leu Phe
   1 5
<210> 70
   <211> 9
   <212> PRT
   <213> Artificial Sequence
<220>
   <223> Based on Homo sapiens sequence
<220>
   <221> misc_feature
   <222> (1)..(1)
   <223> Xaa is N-MeAsp
<220>
   <221> misc_feature
   <222> (4)..(4)
   <223> Xaa is 3,3-diphenyl-L-alanine
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 70
   Xaa Thr His Xaa Pro Ile Cys Ile Xaa
   1               5
<210> 71
   <211> 9
   <212> PRT
   <213> Artificial Sequence
<220>
   <223> Based on Homo sapiens sequence
<220>
   <221> misc_feature
   <222> (1)..(1)
   <223> Xaa is N-MeAsp
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 71
   Xaa Thr His Xaa Xaa Ile Cys Ile Xaa
   1               5
<210> 72
   <211> 9
   <212> PRT
   <213> Artificial Sequence
<220>
   <223> Based on Homo sapiens sequence
<220>
   <221> misc_feature
   <222> (1)..(1)
   <223> Xaa is N-MeAsp
<220>
   <221> misc_feature
   <222> (4)..(4)
   <223> Xaa is 3,3-diphenyl-L-alanine
<220>
   <221> misc_feature
   <222> (6)..(6)
   <223> Xaa is Ach
<220>
   <221> misc_feature
   <222> (8)..(8)
   <223> Xaa is Ach
<220>
   <221> misc_feature
   <222> (9)..(9)
   <223> Having palmitoyl-PEG-miniPEG3- bound thereto
<400> 72
   Xaa Thr His Xaa Pro Xaa Cys Xaa Phe
   1               5
<210> 73
   <211> 9
   <212> PRT
   <213> Artificial Sequence
<220>
   <223> Based on Homo sapiens sequence
<220>
   <221> misc_feature
   <222> (1)..(1)
   <223> Xaa is N-MeAsp
<220>
   <221> misc_feature
   <222> (4)..(4)
   <223> Xaa is 3,3-diphenyl-L-alanine
<220>
   <221> misc_feature
   <222> (5)..(5)
   <223> Xaa is octahydroindole-2-carboxylic acid
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 73
   Xaa Thr His Xaa Xaa Arg Cys Arg Xaa
   1               5
<210> 74
   <211> 9
   <212> PRT
   <213> Artificial Sequence
<220>
   <223> Based on Homo sapiens sequence
<220>
   <221> misc_feature
   <222> (1)..(1)
   <223> Xaa is N-MeAsp
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 74
   Xaa Thr His Xaa Xaa Arg Cys Arg Xaa
   1               5
<210> 75
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 75
   Xaa Thr His Xaa Pro Ile Cys Ile Xaa
   1               5
<210> 76
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 76
   Xaa Thr His Xaa Xaa Ile Cys Ile Xaa
   1               5
<210> 77
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (6)..(6)
   <223> Xaa is Ach
<220>
   <221> misc_feature
   <222> (8)..(8)
   <223> Xaa is Ach
<220>
   <221> misc_feature
   <222> (9)..(9)
   <223> Having palmitoyl-PEG-miniPEG3- bound thereto
<400> 77
   Xaa Thr His Xaa Pro Xaa Cys Xaa Phe
   1               5
<210> 78
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 78
   Xaa Thr His Xaa Xaa Arg Cys Arg Xaa
   1               5
<210> 79
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (6)..(6)
   <223> D-amino acid
<220>
   <221> misc_feature
   <222> (8)..(8)
   <223> D-amino acid
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 79
   Xaa Thr His Xaa Xaa Arg Cys Arg Xaa
   1               5
<210> 80
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (6)..(6)
   <223> Xaa is N-MeArg
<220>
   <221> misc_feature
   <222> (8)..(8)
   <223> Xaa is N-MeArg
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 80
   Xaa Thr His Xaa Xaa Xaa Cys Xaa Xaa
   1               5
<210> 81
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (6)..(6)
   <223> Xaa is bhArg
<220>
   <221> misc_feature
   <222> (8)..(8)
   <223> Xaa is bhArg
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 81
   Xaa Thr His Xaa Xaa Xaa Cys Xaa Xaa
   1 5
<210> 82
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Having palmitoyl-PEG- bound thereto
<220>
   <221> misc_feature
   <222> (9)..(9)
   <223> Xaa is beta-homophenylalanine
<400> 82
   Xaa Thr His Xaa Xaa Arg Cys Arg Xaa
   1 5
<210> 83
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Xaa is beta-homophenylalanine
<400> 83
   Xaa Thr His Xaa Xaa Arg Cys Arg Xaa
   1               5
<210> 84
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (7)..(7)
   <223> Xaa is Cys(S-S-Pal)
<220>
   <221> misc_feature
   <222> (9)..(9)
   <223> Xaa is beta-homophenylalanine
<400> 84
   Xaa Thr His Xaa Xaa Arg Xaa Arg Xaa
   1               5
<210> 85
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (7)..(7)
   <223> Xaa is Cys(S-S-cysteamine-Pal)
<220>
   <221> misc_feature
   <222> (9)..(9)
   <223> Xaa is beta-homophenylalanine
<400> 85
   Xaa Thr His Xaa Xaa Arg Xaa Arg Xaa
   1               5
<210> 86
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (7)..(7)
   <223> Xaa is Cys(S-S-Cys-NHPal)
<220>
   <221> misc_feature
   <222> (9)..(9)
   <223> Xaa is beta-homophenylalanine
<400> 86
   Xaa Thr His Xaa Xaa Arg Xaa Arg Xaa
   1               5
<210> 87
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (7)..(7)
   <223> Xaa is Cys(S-S-Cys)
<220>
   <221> misc_feature
   <222> (9)..(9)
   <223> Having palmitoyl-PEG-miniPEG3- bound thereto
<220>
   <221> misc_feature
   <222> (9)..(9)
   <223> Xaa is beta-homophenylalanine
<400> 87
   Xaa Thr His Xaa Xaa Arg Xaa Arg Xaa
   1               5
<210> 88
   <211> 9
   <212> PRT
   <213> Artificial Sequence
<220>
   <223> Based on Homo sapiens sequence
<220>
   <221> misc_feature
   <222> (1)..(1)
   <223> Xaa is Ida(NHPal)
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Xaa is beta-homophenylalanine
<400> 88
   Xaa Thr His Xaa Xaa Arg Cys Arg Xaa
   1               5
<210> 89
   <211> 10
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Xaa is beta-homophenylalanine
<220>
   <221> misc_feature
   <222> (10)..(10)
   <223> Xaa is Ida(NHPal)
<400> 89
   Xaa Thr His Xaa Xaa Arg Cys Arg Xaa Xaa
   1               5                   10
<210> 90
   <211> 10
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Xaa is beta-homophenylalanine
<220>
   <221> misc_feature
   <222> (10)..(10)
   <223> Xaa is Ahx-Ida(NHPal)
<400> 90
   Xaa Thr His Xaa Xaa Arg Cys Arg Xaa Xaa
   1               5                   10
<210> 91
   <211> 10
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Xaa is beta-homophenylalanine
<220>
   <221> misc_feature
   <222> (10)..(10)
   <223> Xaa is Ahx-Ida(NBzl2)
<400> 91
   Xaa Thr His Xaa Xaa Arg Cys Arg Xaa Xaa
   1               5                   10
<210> 92
   <211> 9
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (9)..(9)
   <223> Having C16 bound thereto
<220>
   <221> misc_feature
   <222> (9)..(9)
   <223> Xaa is beta-homophenylalanine
<400> 92
   Xaa Thr His Xaa Xaa Arg Cys Arg Xaa
   1 5
<210> 93
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (7)..(7)
   <223> Xaa is Cys(S-S-tBut)
<220>
   <221> misc_feature
   <222> (9)..(9)
   <223> Having butanoyl-PEG11- bound thereto
<400> 93
   Asp Thr His Phe Pro Arg Xaa Arg Trp
   1 5
<210> 94
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (7)..(7)
   <223> Xaa is Cys(S-S-tBut)
<220>
   <221> misc_feature
   <222> (9)..(9)
   <223> Having octanoyl-PEG11- bound thereto
<400> 94
   Asp Thr His Phe Pro Arg Xaa Arg Trp
   1               5
<210> 95
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (7)..(7)
   <223> Xaa is Cys(S-S-tBut)
<220>
   <221> misc_feature
   <222> (9)..(9)
   <223> Having palmitoyl-PEG11- bound thereto
<400> 95
   Asp Thr His Phe Pro Arg Xaa Arg Trp
   1               5
<210> 96
   <211> 9
   <212> PRT
   <213> Artificial Sequence
<220>
   <223> Based on Homo sapiens sequence
<220>
   <221> misc_feature
   <222> (1)..(9)
   <223> D-amino acids
<220>
   <221> misc_feature
   <222> (7)..(7)
   <223> Xaa is Cys(S-S-tBut)
<220>
   <221> misc_feature
   <222> (9)..(9)
   <223> Having tetracosanoyl-PEG11- bound thereto
<400> 96
   Asp Thr His Phe Pro Arg Xaa Arg Trp
   1 5
<210> 97
   <211> 10
   <212> PRT
   <213> Artificial Sequence
<220>
   <223> Based on Homo sapiens sequence
<220>
   <221> misc_feature
   <222> (1)..(1)
   <223> Xaa is Ida
<220>
   <221> misc_feature
   <222> (4)..(4)
   <223> Xaa is 3,3-diphenyl-L-alanine
<220>
   <221> misc_feature
   <222> (5)..(5)
   <223> Xaa is L-beta-homoproline
<220>
   <221> misc_feature
   <222> (10)..(10)
   <223> Xaa is Ahx-Ida(NHPal)
<400> 97
   Xaa Thr His Xaa Xaa Arg Cys Arg Trp Xaa
   1 5 10
<210> 98
   <211> 9
   <212> PRT
   <213> Artificial Sequence
<220>
   <223> Based on Homo sapiens sequence
<400> 98
   Asp Thr His Phe Pro Arg Cys Arg Asp
   1 5
<210> 99
   <211> 8
   <212> PRT
   <213> Artificial Sequence
<220>
   <223> Based on Homo sapiens sequence
<400> 99
   Asp Thr His Phe Pro Arg Cys Arg
   1 5
<210> 100
   <211> 9
   <212> PRT
   <213> Artificial Sequence
<220>
   <223> Based on Homo sapiens sequence
<220>
   <221> misc_feature
   <222> (1)..(9)
   <223> D-amino acids
<400> 100
   Asp Thr His Phe Pro Arg Cys Arg Asp
   1 5
<210> 101
   <211> 8
   <212> PRT
   <213> Artificial Sequence
<220>
   <223> Based on Homo sapiens sequence
<220>
   <221> misc_feature
   <222> (1)..(8)
   <223> D-amino acids
<400> 101
   Asp Thr His Phe Pro Arg Cys Arg
   1 5

## Description

### CROSS-REFERENCE TO RELATED APPLICATIONS

This application claims the benefit of U.S. Application No. 62/097,429, filed December 29, 2014.

### ACKNOWLEDGEMENT OF GOVERNMENT SUPPORT

This invention was made with Government support under DK090554, awarded by the National Institutes of Health. The Government has certain rights in the invention.

### REFERENCE TO A SEQUENCE LISTING SUBMITTED VIA EFS-WEB

The content of the ASCII text file of the sequence listing named "20151227_034044_155W01_seq_ST25" which is 41.7 kb in size was created on December 27, 2015 and electronically submitted via EFS-Web is part of the description.

### BACKGROUND OF THE INVENTION

### 1. FIELD OF THE INVENTION

The present invention generally relates to S-alkylated hepcidin peptides and methods of making and using thereof.

### 2. DESCRIPTION OF THE RELATED ART

Hepcidin, a peptide hormone produced by the liver, is a regulator of iron homeostasis in humans and other mammals. Hepcidin acts by binding to its receptor, the iron export channel ferroportin, and causing its internalization and degradation. Human hepcidin is a 25-amino acid peptide (Hep25). See Krause et al. (2000) FEBS Lett 480:147-150, and Park et al. (2001) J Biol Chem 276:7806-7810. The structure of the bioactive 25-amino acid form of hepcidin is a simple hairpin with 8 cysteines that form 4 disulfide bonds as described by Jordan et al. (2009) J Biol Chem 284:24155-67. The N terminal region is required for iron-regulatory function, and deletion of 5 N-terminal amino acid residues results in a loss of iron-regulatory function. See Nemeth et al. (2006) Blood 107:328-33.

Abnormal hepcidin activity is associated with iron overload diseases which include hereditary hemochromatosis and iron-loading anemias and myelodysplasia. Hereditary hemochromatosis (HH) is a genetic iron overload disease that is mainly caused by hepcidin deficiency, or very rarely by hepcidin resistance. This allows excessive absorption of iron from the diet and development of iron overload. Clinical manifestations of HH may include liver disease (hepatic cirrhosis, hepatocellular carcinoma), diabetes, and heart failure. Currently, the only treatment for HH is regular phlebotomy, which is effective but very burdensome for the patients.

Iron-loading anemias are hereditary anemias with ineffective erythropoiesis such as β-thalassemia, which are accompanied by severe iron overload. Complications from iron overload are the main cause of morbidity and mortality for these patients. Hepcidin deficiency is the main cause of iron overload in untransfused patients, and contributes to iron overload in transfused patients. The current treatment for iron overload in these patients is iron chelation which is very burdensome, sometimes ineffective and accompanied by frequent side effects.

Mini-hepcidin peptides disclosed in WO 2010/065815 and modified mini-hepcidin peptides disclosed in WO 2013/086143 exhibit hepcidin activity and can be used to modulate iron metabolism and treat diseases of iron metabolism. Many of these mini-hepcidin peptides contain an unprotected free-cysteine residue, e.g., at the A7 amino acid position. Unfortunately, peptide-based therapeutics that contain and/or release free sulfhydryl group(s) can be problematic as they may exhibit (1) decreased stability associated with inherent free-thiol reactivity (S-alkylation/oxidation), and/or (2) dermatological side effects (e.g. skin eruptions).

### SUMMARY OF THE INVENTION

In some embodiments, the present invention is directed to an S-alkylated hepcidin peptide comprising or consisting of the following Structural Formula IA or IB

A1-A2-A3-A4-A5-A6-A7-A8-A9-A10 IA

A10-A9-A8-A7-A6-A5-A4-A3-A2-A1 IB

wherein
A1 is Asp, D-Asp, Glu, D-Glu, pyroglutamate, D-pyroglutamate, Gln, D-Gln, Asn, D-Asn, bhAsp, Ida, Ida(NHPal), or N-MeAsp;
A2 is Thr, D-Thr, Ser, D-Ser, Val, D-Val, Ile, D-Ile, Ala, D-Ala, Tie, Inp, Chg, bhThr, or N-MeThr;
A3 is His, D-His, Asn, D-Asn, Arg, D-Arg, L-His(π-Me), D-His(π-Me), L-His(τ-Me), or D-His(τ-Me);
A4 is Phe, D-Phe, Leu, D-Leu, Ile, D-Ile, Trp, D-Trp, Tyr, D-Tyr, Phg, bhPhe, Dpa, Bip, 1Nal, 2Nal, bhDpa, Amc, PheF5, hPhe, Igl, or cyclohexylalanine;
A5 is Pro, D-Pro, Ser, D-Ser, Oic, bhPro, trans-4-PhPro, cis-4-PhPro, cis-5-PhPro, or Idc,;
A6 is Arg, D-Arg, Ile, D-Ile, Leu, D-Leu, Thr, D-Thr, Lys, D-Lys, Val, D-Val, D-Nω,ω-dimethyl-arginine, L-Nω,ω-dimethyl-arginine, D-homoarginine, L-homoarginine, D-norarginine, L-norarginine, citrulline, a modified Arg wherein the guanidinium group is modified or substituted, Norleucine, norvaline, bhIle, Ach, N-MeArg, or N-Melle;
A8 is Arg, D-Arg, Ile, D-Ile, Leu, D-Leu, Thr, D-Thr, Lys, D-Lys, Val, D-Val, D-Nω,ω-dimethyl-arginine, L-Nω,ω-dimethyl-arginine, D-homoarginine, L-homoarginine, D-norarginine, L-norarginine, citrulline, a modified Arg wherein the guanidinium group is modified or substituted, Norleucine, norvaline, bhIle, Ach, N-MeArg, or N-Melle;
A9 is Phe, D-Phe, Leu, D-Leu, Ile, D-Ile, Tyr, D-Tyr, Trp, D-Trp, Phe-R^{a}, D-Phe-R^{a}, Dpa-R^{a}, D-Dpa-R^{a}, Trp-R^{a}, bhPhe-R^{a}, PheF5, N-MePhe, benzylamide, 2-aminoindane, bhPhe, Dpa, Bip, 1Nal, 2Nal, bhDpa, or cyclohexylalanine, which may or may not have R^{a} linked thereto, wherein R^{a} is palmitoyl-PEG-, wherein PEG is PEG11 or miniPEG3, palmitoyl-PEG-PEG, wherein PEG is PEG11 or miniPEG3, butanoyl (C4)-PEG11-, octanoyl (C8, Caprylic)-PEG11-, palmitoyl (C16)-PEG11-, or tetracosanoyl (C24, Lignoceric)-PEGll-; and
A10 is Cys, D-Cys, Ser, D-Ser, Ala, D-Ala, Ida, Ida (NHPal)Ahx, or Ida(NBzl2)Ahx; and A7 has Structural Formula A: 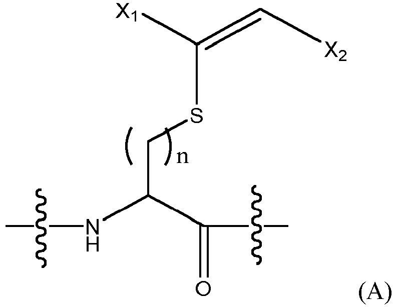
wherein
n is 1 or 2 and one or more of the hydrogens bonded to the Cn atom(s) may be substituted with a (C₁-C₃)alkyl,
X₁ and X₂ are each independently selected from the group consisting of H, alkyl, alkoxy, alkoxycarbonyl, cycloalkyl, aryl, heteroaryl, heterocycloalkyl, acyl, sulfonyl, alkylsulfonyl, alkylamino, alkylaminocarbonyl, dialkylaminocarbonyl, carboxyl, carbamoyl and 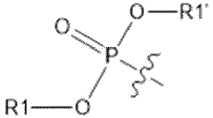 wherein R1 and R1' are each independently selected from the group consisting of H, methyl, (C₂)alkyl, (C₃)alkyl, (C₄)alkyl, (C₁-C₅)alkyl, (C₆)alkyl, (C₇)alkyl, (C₈)alkyl, (C₉)alkyl, and (C₁₀)alkyl;
wherein the carboxy-terminal amino acid is in amide or carboxy- form; and wherein A1, A1 to A2, A10, or a combination thereof are optionally absent. In some embodiments, the S-alkylated hepcidin peptide of the present invention has an amino acid sequence selected from SEQ ID NOs: 1-101. In some embodiments, the amino acid residue having Structural Formula A corresponds to a thiol containing amino acid of SEQ ID NOs: 1-101. In some embodiments, A1 is Ida, A2 is Thr, A3 is His, A4 is Dpa, A5 is bhPro, A6 is Arg, A8 is Arg, A9 is bhPhe, and A10 is Ahx-Ida (NHPal). In some embodiments, X₁ and X₂, are each independently selected from the group consisting of H, phenyl, 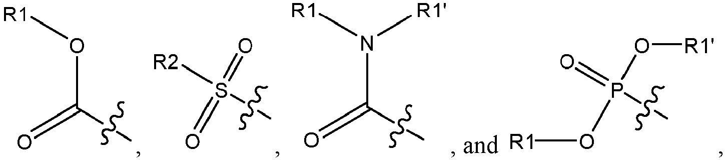 wherein R1 and R1' are each independently selected from the group consisting of H, methyl, (C₂)alkyl, (C₃)alkyl, (C₄)alkyl, (C₁-C₅)alkyl, (C₆)alkyl, (C₇)alkyl, (C₈)alkyl, (C₉)alkyl, and (C₁₀)alkyl; and R2 is -NR1R1', methyl, (C₂)alkyl, (C₃)alkyl, (C₄)alkyl, (C₁-C₅)alkyl, (C₆)alkyl, (C₇)alkyl, (C₈)alkyl, (C₉)alkyl, and (C₁₀)alkyl. In some embodiments, R1 and R1' are each independently selected from the group consisting of H, methyl, ethyl, isopropyl, and tert-butyl. In some embodiments, X₁ and X₂ are each independently selected from the group consisting of H, phenyl, 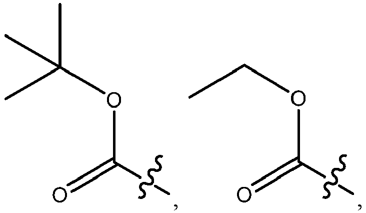 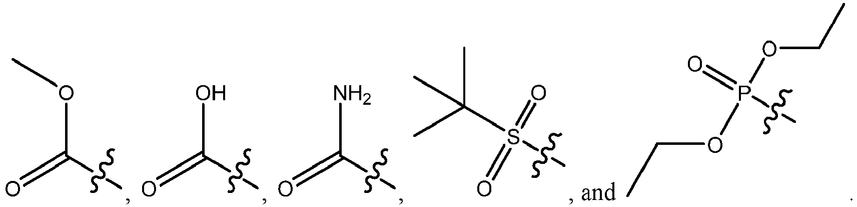 In some embodiments, X₁ and X₂ are (a) both 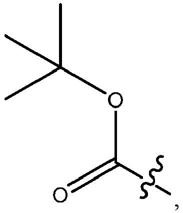 (b) both 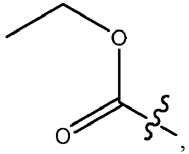 (c) both 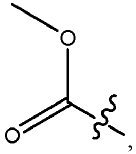 (c) H and 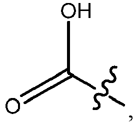 respectively, (d) phenyl and 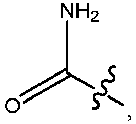 respectively, (e) both 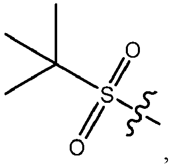 or (f) both

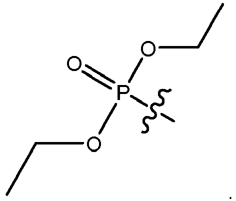

In some embodiments, the present invention is directed to a composition comprising at least one S-alkylated hepcidin peptide of the present invention, e.g., an S-alkylated hepcidin peptide as set forth in paragraph [0016] above.

In some embodiments, the present invention is directed to a method of binding a ferroportin or inducing ferroportin internalization and degradation which comprises contacting the ferroportin with at least one S-alkylated hepcidin peptide of the present invention, e.g., an S-alkylated hepcidin peptide as set forth in paragraph [0016] above, or a composition thereof.

In some embodiments, the present invention is directed to a kit comprising at least one S-alkylated hepcidin peptide of the present invention, e.g., an S-alkylated hepcidin peptide as set forth in paragraph [0016] above, or a composition thereof packaged together with a reagent, a device, instructional material, or a combination thereof.

In some embodiments, the present invention is directed to a complex comprising at least one S-alkylated hepcidin peptide of the present invention, e.g., an S-alkylated hepcidin peptide as set forth in paragraph [0016] above, bound to a ferroportin or an antibody.

The present invention also describes a method of treating a disease of iron metabolism in a subject which comprises administering at least one S-alkylated hepcidin peptide of the present invention, e.g., an S-alkylated hepcidin peptide as set forth in paragraph [0016] above, or a composition thereof to the subject. In some embodiments, the disease of iron metabolism is an iron overload disease. In some embodiments, the present invention is directed to the use of one or more S-alkylated hepcidin peptides of the present invention, e.g., an S-alkylated hepcidin peptide as set forth in paragraph [0016] above, or a composition thereof for the manufacture of a medicament for treating a disease of iron metabolism and/or lowering the amount of iron in a subject in need thereof. In some embodiments, the present invention is directed to one or more S-alkylated hepcidin peptides of the present invention, e.g., an S-alkylated hepcidin peptide as set forth in paragraph [0016] above, or a composition thereof for use in treating a disease of iron metabolism and/or lowering the amount of iron in a subject in need thereof. In some embodiments, the present invention is directed to the use of one or more S-alkylated hepcidin peptides of the present invention, e.g., an S-alkylated hepcidin peptide as set forth in paragraph [0016] above, or a composition thereof for the manufacture of a medicament for treating a disease of iron metabolism and/or lowering the amount of iron in a subject in need thereof, wherein the medicament is prepared to be administered at an effective daily dose as a single daily dose or as divided daily doses. In some embodiments, the effective daily dose is about 10-500 µg/kg/day and the medicament is formulated for subcutaneous injection. In some embodiments, the effective daily dose is about 10-1000 µg/kg/day and the medicament is formulated for oral, pulmonary, or mucosal administration. In some embodiments, the subject is a mammal. In some embodiments, the subject is human.

Both the foregoing general description and the following detailed description are exemplary and explanatory only and are intended to provide further explanation of the invention as claimed. The accompanying drawings are included to provide a further understanding of the invention and are incorporated in and constitute part of this specification, illustrate several embodiments of the invention, and together with the description serve to explain the principles of the invention.

### DESCRIPTION OF THE DRAWINGS

This invention is further understood by reference to the drawings wherein:
Figure 1 schematically shows the synthetic scheme for S-alkylation of hepcidin peptides using PR73 as an example.
Figure 2 shows the general structure of S-derivatized PR73 analogs. The structures in the top row are the structures which replace that encompassed in the circle shown in the bottom structure (PR73 (SEQ ID NO: 90)).
Figures 3A and 3B are graphs comparing the *in vitro* and *in vivo* activity of PR73 and PR73SH. Figure 3A are representative examples of *in vitro* dose response curves obtained for PR73 and PR73SH analogs using ferroportin degradation assay. Figure 3B are bar graphs comparing the *in vivo* activity of PR73 and PR73SH at 6, 24, and 48 hour time-points after administration by intraperitoneal injection.

### DETAILED DESCRIPTION OF THE INVENTION

As used herein, "hepcidin peptides" refers to mini-hepcidin peptides disclosed in WO 2010/065815 and modified mini-hepcidin peptides disclosed in WO 2013/086143. As used herein, a "thiol-containing hepcidin peptide" refers to a hepcidin peptide having an amino acid residue containing a free thiol group (-SH). Thiol-containing hepcidin peptides include those having an unprotected free cysteine residue at amino acid position 7 as set forth in the structural formulas of WO 2010/065815 and WO 2013/086143.

The present invention provides S-alkylated hepcidin peptides and methods of making and using thereof. As used herein, an "S-alkylated hepcidin peptide" refers to a peptide in which the hydrogen of the free thiol group (-SH) of a thiol-containing hepcidin peptide is substituted by S-alkylation.

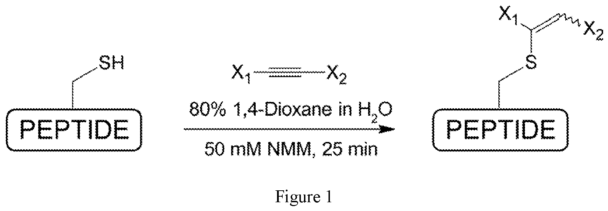

As disclosed herein, 1,2-double substituted vinyl-sulfides, which may be efficiently synthesized from corresponding electron-deficient alkynes and unprotected free-cysteine containing peptides in aqueous media, were used as a protecting moiety. See Figure 1. Specifically, S-alkylated hepcidin peptides, PR73SA-PR73SH, were derived in a one-step reaction from parental peptide, PR73, as a representative thiol-containing hepcidin peptide. PR73 was synthesized as previously described. See Preza, et al. (2011) J. Clin. Invest., 121, 4880. Briefly, PR73 was assembled by the solid phase method using CEM Liberty automatic microwave peptide synthesizer (CEM Corporation Inc., Matthews, NC), applying 9-fluorenylmethyloxycarbonyl (Fmoc) chemistry and commercially available amino acid derivatives and reagents (EMD Biosciences, San Diego, CA and Chem-Impex International, Inc., Wood Dale, IL). Rinkamide- MBHA resin (EMD Biosciences, San Diego, CA) was used as a solid support. Peptide was cleaved from resin using modified reagent K (TFA 94% (v/v); phenol, 2% (w/v); water, 2% (v/v); TIS, 1% (v/v); EDT, 1% (v/v); 2 hours) and precipitated by addition of ice-cold diethyl ether. The peptide was purified by preparative reverse-phase high performance liquid chromatography (RP-HPLC) and its purity evaluated by matrix-assisted laser desorption ionization spectrometry (MALDI-MS) as well as analytical RP-HPLC.

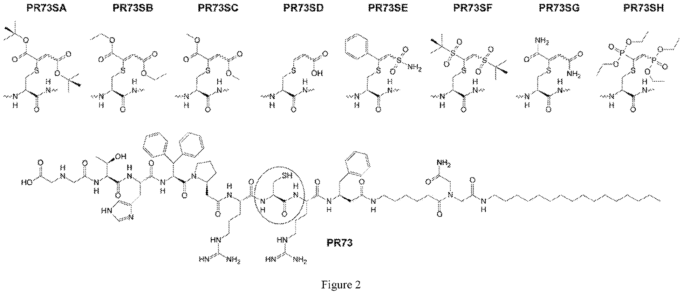

PR73 was solubilized in 80% 1,4-dioxane in water, containing 50 mM N-methylmorpholine (NMM) (about 2 mg/mL) and subsequently a given electron-deficient alkyne was added (2 eq.). The S-alkylated hepcidin peptides as exemplified herein, and the given electron-deficient alkynes used to produce the exemplified S-alkylated hepcidin peptides are: (1) PR73SA - Di-tert-butyl acetylenedicarboxylate, (2) PR73SB - Diethyl acetylenedicarboxylate, (3) PR73SC - Dimethyl acetylenedicarboxylate, (4) PR73SD - Acetylenedicarboxylic acid, (5) PR73SE - 2-Phenylethynesulfonamide (Pifithrin-µ), (6) PR73SF - 1,2-Bis(tert-butylsulfonyl)acetylene, (7) PR73SG - Acetylenedicarboxamide, and (8) PR73SH - Bis(diethoxyphosphoryl)acetylene. Figure 2 shows the chemical structures of the exemplified S-alkylated hepcidin peptides. The mixture was vigorously stirred for 25 minutes at room temperature and subsequently lyophilized. A solid residue was obtained and purified by preparative reverse-phase high performance liquid chromatography (RP-HPLC) and its purity was evaluated by matrix-assisted laser desorption ionization spectrometry (MALDI-MS) as well as analytical RP-HPLC. See Table 1.

| **Table 1** | | | | |
|---|---|---|---|---|
| Analytical and *in vitro* activity data for S-alkylated PR73 analogs | | | | |
| **Peptide** | **Composition** | **MW Calc/Found** | **R_{T} [min]** | **EC₅₀ [nM] TREX-hFpn-GFP cells** |
| PR73 | C₈₆H₁₃₃N₂₁O₁₅S | 1733.19/1734.34 | 47.11 | 4.2±0.3 |
| PR73SA | C₉₈H₁₅₁N₂₁O₁₉S | 1959.46/1959.80 | 52.47 | 6.3±1.2 |
| PR73SB | C₉₄H₁₄₃N₂₁O₁₉S | 1903.35/1904.58 | 49.44 | 10.4±1.2 |
| PR73SC | C₉₂H₁₃₉N₂₁O₁₉S | 1875.30/1876.60 | 48.32 | 12.6±1.8 |
| PR73SD | C₈₉H₁₃₅N₂₁O₁₇S | 1803.24/1803.66 | 46.60 | 218.1±13.4 |
| PR73SE | C₉₄H₁₄₀N₂₂O₁₇S₂ | 1914.40/1915.02 | 48.52 | 34.0±5.4 |
| PR73SF | C₉₆H₁₅₁N₂₁O₁₉S₃ | 1999.56/1999.80 | 52.89 * | 10.0±3.4 |
| PR73SG | C₉₀H₁₃₇N₂₃O₁₇S | 1845.28/1846.59 | 49.33 * | 8.4±2.5 |
| PR73SH | C₉₆H₁₅₃N₂₁O₂₁P₂S | 2031.40/2031.33 | 52.18 * | 1.1±0.1 |
| Analytical RP-HPLC was performed using an analytical reversed-phase C4 XBridge™ BEH300 column, 4.6 x 150 mm, 3.5 µm (Waters, Milford, MA), or (*) an analytical reversed-phase C18 Symmetry Shield™ column, 4.6 x 250 mm, 5 µm (Waters, Milford, MA). | | | | |

The S-alkylated hepcidin peptides were tested *in vitro* using a previously described cellular assay based on Fpn degradation. See e.g., Nemeth, et al. (2006) Blood 107: 328. Briefly, HEK293:TREX-Fpn-GFP, a cell line stably transfected with the human ferroportin-GFP construct under the control of doxycycline-inducible promoter, was plated on poly-D-lysine-coated plates in the presence of 20 µM FAC. Fpn expression was induced with 500 ng/mL doxycycline treatment for 24 hours. Then, doxycycline was washed off, and cells were treated with peptides for 24 hours. Cells were then trypsinized and resuspended at 1 x 10⁶ cells/mL, and the intensity of green fluorescence was analyzed by flow cytometry using FACScan (fluorescence activated cell scanner) Analytic Flow Cytometer (Becton Dickinson, San Jose, CA) with CellQuest version 3.3 software. Cells not induced with doxycycline to express Fpn-GFP were used to establish a gate to exclude background fluorescence. Cells induced with doxycycline, but not treated with any peptides, were used as the positive control. Each peptide treatment was repeated independently 3 to 6 times. The results were expressed as a fraction of the activity of Hep25, according to Formula 1, (Fx - FHep25)/(Funtreated - FHep25), where F is the mean of the gated green fluorescence and x is the peptide. The results are summarized in Table 1. Generally, the S-alkylated hepcidin peptides showed high potency in the low nanomolar range. PR73SH, however, showed bioactivity (EC₅₀ = 1.1 ± 0.1 nM) that is higher than the parental PR73 (EC₅₀ = 4.2 ± 0.3 nM). Interestingly, the chemical synthesis of the S-alkylated hepcidin peptides does not appear to have a significant impact on bioactivity, rather the overall steric hindrance plays a significant role, with the most bulky substituents having hepcidin activity that is the same or better than Hep25. Hydrophobicity may also play a role, as activity increases in the carboxy-esters-substituent(s) order: -CH3 < -C₂H₅ < -C₄H₉ (PR73SC < PR73SB < PR73SA).

Additionally, the geometry of the vinyl substituents (planar versus tetrahedral) does not appear to significantly influence activity, as planar analog PR73SA has fairly similar potency to its tetragonal counterpart (PR73 SF). Considering that remaining tetragonal analog PR73SH shows highest activity, and the fact that all 3 analogs (e.g., PR73SA, PR73SF and PR73SH) are chemically fairly similar having the same number of substituent(s)-carbon-atoms (2 x 4 = 8), overall volume/space occupied by S-attached moiety appears again as important factor, with the activity increasing from most compact (PR73 SF) to most bulky (PR73SH) substituent(s). Consistently, PR73SD, which has the most hydrophilic and least bulky substituent, shows the lowest potency (EC₅₀ = 218.1 ± 13.4 nM).

Based on *in vitro* results, PR73SH was selected as a suitable candidate for animal studies, which were carried out as previously described. See Preza, et al. (2011) J. Clin. Invest. 121:4880; Ramos, et al. (2012) Blood 120:3829; and Nemeth, et al. (2006) Blood 107:328. Animal studies were approved by the Animal Research Committee at UCLA. Briefly, C57BL/6 mice were obtained from The Jackson Laboratory (Bar Harbor, ME) and were maintained on NIH 31 rodent diet (iron content 336 mg/kg; Harlan Teklad, Indianapolis, IA). Mice were injected intraperitoneally either with 100 µL PBS (control) or with 50 or 100 nmoles peptide in 100 µL PBS. Mice were killed 6, 24, and 48 hours later, blood was collected by cardiac puncture, and serum was separated using Microtainer tubes (Becton Dickinson, Franklin Lakes, NJ). Serum iron was determined by using a colorimetric assay (Diagnostic Chemicals, Oxford, CT), which was modified for the microplate format so that 50 µL serum was used per measurement. See Nemeth, et al. (2004) J. Clin. Invest. 113(9): 1271-1276. The results were expressed as the percentage of decrease in serum iron when compared with the average value of serum iron levels in PBS-injected mice.

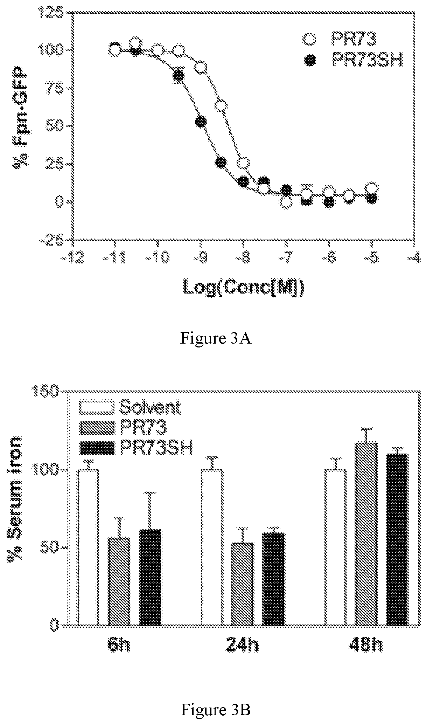

*In vivo* activity of PR73SH and PR73 was compared by assaying serum iron levels at 3 time points: (6, 24, and 48 hours) and concentrations that were previously shown to be sufficient for PR73 to exert potent bioactivity (50-100 nmoles/mouse). PR73SH activity was similar to the parental PR73 activity profile, with decreased serum iron observed at 6 and 24 hour time points, but not at the 48 hour time point (Figure 3B). Since no significant activity difference between PR73 and PR73SH was observed in either, *in vitro* or *in vivo* experiments, S-alkylated hepcidin peptides may be used to diseases of iron metabolism, such as iron overload disease, in subjects.

Therefore, in some embodiments, the S-alkylated hepcidin peptides according to the present invention comprise an S-alkylated cysteine residue having the bracketed structure set forth in Structural Formula I: 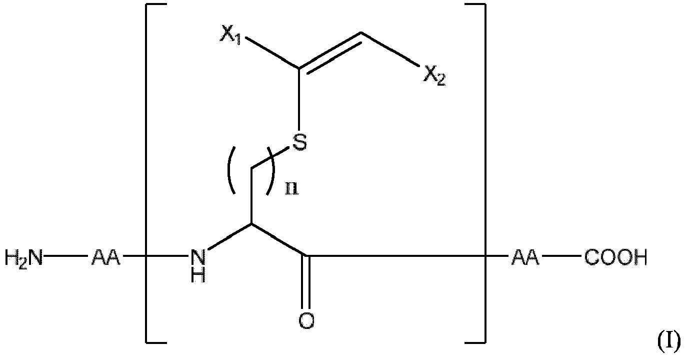 wherein n is 1 or 2 and one or more of the hydrogens bonded to the Cn atom(s) may be substituted with a (C₁-C₃)alkyl, AA represent the amino acid residues flanking the bracketed S-alkylated cysteine residue (in brackets) and X₁ and X₂, may be the same or different, and are the X₁ and X₂ groups of an electron-deficient alkyne having the formula 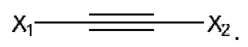 In some embodiments X₁ and X₂ are each independently selected from the group consisting of H, alkyl, alkoxy, alkoxycarbonyl, cycloalkyl, aryl, heteroaryl, heterocycloalkyl, acyl, sulfonyl, alkylsulfonyl, alkylamino, alkylaminocarbonyl, dialkylaninocarbonyl, carboxyl, and carbamoyl. In some embodiments, X₁ and X₂, are each independently selected from the group consisting of H, phenyl, 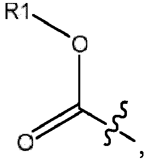 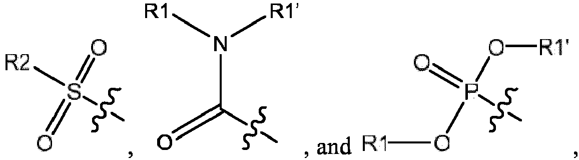 wherein R1 and R1' are each independently selected from the group consisting of H, methyl, (C₂)alkyl, (C₃)alkyl, (C₄)alkyl, (C₁-C₅)alkyl, (C₆)alkyl, (C₇)alkyl, (C₈)alkyl, (C₉)alkyl, and (C₁₀)alkyl; and R2 is -NR1R1', methyl, (C₂)alkyl, (C₃)alkyl, (C₄)alkyl, (C₁-C₅)alkyl, (C₆)alkyl, (C₇)alkyl, (C₈)alkyl, (C₉)alkyl, or (C₁₀)alkyl. In some embodiments, R1 and R1' are each independently selected from the group consisting of H, methyl, ethyl, isopropyl, and tert-butyl. In some embodiments, the S-alkylated cysteine residue is at amino acid position 7 corresponding to the structural formulas of WO 2010/065815 and WO 2013/086143.

In some embodiments, the S-alkylated hepcidin peptides according to the present invention have the following Structural Formula IA or IB

A1-A2-A3-A4-A5-A6-A7-A8-A9-A10 IA

A10-A9-A8-A7-A6-A5-A4-A3-A2-A1 IB

wherein
A1 is Asp, D-Asp, Glu, D-Glu, pyroglutamate, D-pyroglutamate, Gln, D-Gln, Asn, D-Asn, bhAsp, Ida, Ida(NHPal), or N-MeAsp;
A2 is Thr, D-Thr, Ser, D-Ser, Val, D-Val, Ile, D-Ile, Ala, D-Ala, Tie, Inp, Chg, bhThr, or N-MeThr;
A3 is His, D-His, Asn, D-Asn, Arg, D-Arg, L-His(π-Me), D-His(π-Me), L-His(τ-Me), or D-His(τ-Me);
A4 is Phe, D-Phe, Leu, D-Leu, Ile, D-Ile, Trp, D-Trp, Tyr, D-Tyr, Phg, bhPhe, Dpa, Bip, 1Nal, 2Nal, bhDpa, Amc, PheF5, hPhe, Igl, or cyclohexylalanine;
A5 is Pro, D-Pro, Ser, D-Ser, Oic, bhPro, trans-4-PhPro, cis-4-PhPro, cis-5-PhPro, or Idc,;
A6 is Arg, D-Arg, Ile, D-Ile, Leu, D-Leu, Thr, D-Thr, Lys, D-Lys, Val, D-Val, D-Nω,ω-dimethyl-arginine, L-Nω,ω-dimethyl-arginine, D-homoarginine, L-homoarginine, D-norarginine, L-norarginine, citrulline, a modified Arg wherein the guanidinium group is modified or substituted, Norleucine, norvaline, bhIle, Ach, N-MeArg, or N-Melle;
A8 is Arg, D-Arg, Ile, D-Ile, Leu, D-Leu, Thr, D-Thr, Lys, D-Lys, Val, D-Val, D-Nω,ω-dimethyl-arginine, L-Nω,ω-dimethyl-arginine, D-homoarginine, L-homoarginine, D-norarginine, L-norarginine, citrulline, a modified Arg wherein the guanidinium group is modified or substituted, Norleucine, norvaline, bhIle, Ach, N-MeArg, or N-Melle;
A9 is Phe, D-Phe, Leu, D-Leu, Ile, D-Ile, Tyr, D-Tyr, Trp, D-Trp, Phe-R^{a}, D-Phe-R^{a}, Dpa-R^{a}, D-Dpa-R^{a}, Trp-R^{a}, bhPhe-R^{a}, PheF5, N-MePhe, benzylamide, 2-aminoindane, bhPhe, Dpa, Bip, 1Nal, 2Nal, bhDpa, or cyclohexylalanine, which may or may not have R^{a} linked thereto, wherein R^{a} is palmitoyl-PEG-, wherein PEG is PEG11 or miniPEG3, palmitoyl-PEG-PEG, wherein PEG is PEG11 or miniPEG3, butanoyl (C4)-PEG11-, octanoyl (C8, Caprylic)-PEG11-, palmitoyl (C16)-PEG11-, or tetracosanoyl (C24, Lignoceric)-PEG11-; and
A10 is Cys, D-Cys, Ser, D-Ser, Ala, D-Ala, Ida, Ida(NHPal)Ahx, or Ida(NBzl2)Ahx; and A7 has Structural Formula A: 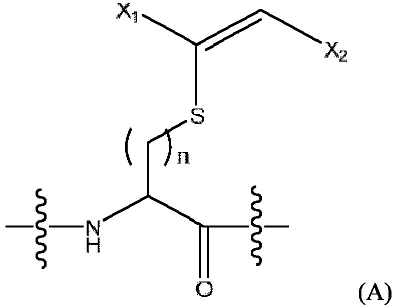 wherein
   n is 1 or 2 and one or more of the hydrogens bonded to the Cn atom(s) may be substituted with a (C₁-C₃)alkyl,
   X₁ and X₂ are each independently selected from the group consisting of H, alkyl, alkoxy, alkoxycarbonyl, cycloalkyl, aryl, heteroaryl, heterocycloalkyl, acyl, sulfonyl, alkylsulfonyl, alkylamino, alkylaminocarbonyl, dialkylaninocarbonyl, carboxyl, and carbamoyl;
   wherein the carboxy-terminal amino acid is in amide or carboxy- form; and
   wherein A1, A1 to A2, A10, or a combination thereof are optionally absent. In some embodiments, X₁ and X₂, are each independently selected from the group consisting of H, phenyl, 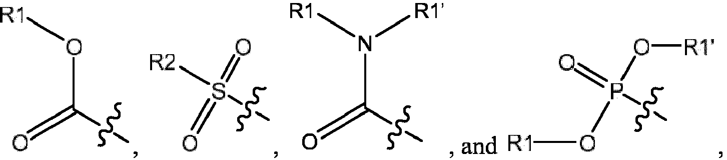 wherein R1 and R1' are each independently selected from the group consisting of H, methyl, (C₂)alkyl, (C₃)alkyl, (C₄)alkyl, (C₁-C₅)alkyl, (C₆)alkyl, (C₇)alkyl, (C₈)alkyl, (C₉)alkyl, and (C₁₀)alkyl; and R2 is -NR1R1', methyl, (C₂)alkyl, (C₃)alkyl, (C₄)alkyl, (C₁-Cs)alkyl, (C₆)alkyl, (C₇)alkyl, (C₈)alkyl, (C₉)alkyl, or (C₁₀)alkyl. In some embodiments, R1 and R1' are each independently selected from the group consisting of H, methyl, ethyl, isopropyl, and tert-butyl. In some embodiments, amino acid residue having Structural Formula A is A7.

As provided herein, "Cn atom(s)" refers to the carbon atom(s) in the parentheticals of the Structural Formulas I and A herein. Thus, an example of Structural Formula A having "one or more of the hydrogens bonded to the Cn atom(s) may be substituted with a (C₁-C₃)alkyl" is 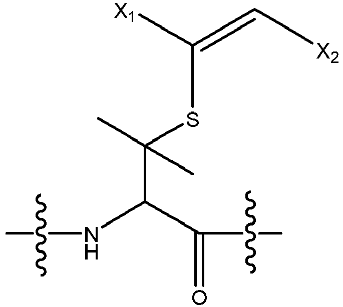 where n is 1 and both the hydrogens are replaced with methyl.

In some embodiments, an S-alkylated hepcidin peptide according to the present invention is a hepcidin peptide having at least one amino acid residue substituted with a residue having Structural Formal A as set forth above, wherein said hepcidin peptides are selected from Table 2, Table 3, and Table 4.

In some embodiments, the amino acid residue, of the hepcidin peptides of Table 2, Table 3, or Table 4, which is substituted with a residue having Structural Formal A is the residue at amino acid position 7. In some embodiments, the amino acid residue, of the hepcidin peptides of Table 2, Table 3, or Table 4, which is substituted with a residue having Structural Formal A is a thiol containing amino acid residue.

The uncommon and unnatural amino acids referenced herein are provided in Table 5.

| **Table 2** | | | | | | | | | | |
|---|---|---|---|---|---|---|---|---|---|---|
| **Name** | **1** | **2** | **3** | **4** | **5** | **6** | **7** | **8** | **9** | **10** |
| Hep25 DTHFPICIFCCGCCHRSKCGMCCKT (SEQ ID NO: 1) | | | | | | | | | | |
| Hep10wt (SEQ ID NO: 2) | D | T | H | F | P | I | C | I | F | C |

| **Length** | | | | | | | | | | |
|---|---|---|---|---|---|---|---|---|---|---|
| Hep4 (Hep4-7) (SEQ ID NO: 3) | - | - | - | F | P | I | C | - | - | - |
| Hep5 (Hep3-7) (SEQ ID NO: 4) | - | - | H | F | P | I | C | - | - | - |
| Hep6 (Hep3-8) (SEQ ID NO: 5) | - | - | H | F | P | I | C | I | - | - |
| Hep7ΔDT (Hep3-9) (SEQ ID NO: 6) | - | - | H | F | P | I | C | I | F | - |
| Hep7 (Hep1-7) (SEQ ID NO: 7) | D | T | H | F | P | I | C | - | - | - |
| Hep8 (Hep1-8) (SEQ ID NO: 8) | D | T | H | F | P | I | C | I | - | - |
| Hep9 (Hep1-9) (SEQ ID NO: 9) | D | T | H | F | P | I | C | I | F | - |
| Hep10 (Hep1-10 C7A) (SEQ ID NO: 10) | D | T | H | F | P | I | A | I | F | C |

| **Thiol Modified** | | | | | | | | | | |
|---|---|---|---|---|---|---|---|---|---|---|
| Hep9F4A (SEQ ID NO: 11) | D | T | H | A | P | I | C | I | F | - |
| Hep9C7-SStBut (SEQ ID NO: 12) | D | T | H | A | P | I | C-S-tBut | I | F | - |
| Hep9C7-tBut (SEQ ID NO: 13) | D | T | H | A | P | I | C-tBut | I | F | - |
| Hep9-C7A (SEQ ID NO: 14) | D | T | H | F | P | I | A | I | F | - |
| Hep9-C7S (SEQ ID NO: 15) | D | T | H | F | P | I | S | I | F | - |
| (D)C (SEQ ID NO: 16) | D | T | H | F | P | I | C | I | F | - |
| homoC (SEQ ID NO: 17) | D | T | H | F | P | I | homoCys | I | F | - |
| ^{Pen} (SEQ ID NO: 18) | D | T | H | F | P | I | Pen | I | | F |
| (D)Pen (SEQ ID NO: 19) | D | T | H | F | P | I | (D)Pen | I | | F |
| Dap(AcBr) (SEQ ID NO: 20) | D | T | H | F | P | I | Dap(AcBr) | I | | F |

| **Unnatural AA's** | | | | | | | | | | |
|---|---|---|---|---|---|---|---|---|---|---|
| PR10 (SEQ ID NO: 21) | D | Tle | H | Phg | Oic | Chg | C | Chg | | F |
| PR11 (SEQ ID NO: 22) | D | Tle | H | P | Oic | Chg | C | Chg | | F |

| **Retroinverted** | | | | | | | | | | |
|---|---|---|---|---|---|---|---|---|---|---|
| PR12 (SEQ ID NO: 23) | F | I | C | I | P | F | H | T | | D |
| hHep7ΔDT (SEQ ID NO: 24) | F | I | C | I | P | F | H | - | | - |

| Modified **Retroinverted** | | | | | | | | | | |
|---|---|---|---|---|---|---|---|---|---|---|
| PR23 (SEQ ID NO: 25) | R2-F | I | C | I | P | F | H | T | | D |
| PR24 (SEQ ID NO: 26) | R3-F | I | C | I | P | F | H | T | | D |
| PR25 (SEQ ID NO: 27) | F | I | C | I | P | F | H | T | | D-R6 |
| PROC (SEQ ID NO: 28) | F | I | C | I | P | F | H | T | | D-R7 |
| PR27 (SEQ ID NO: 29) | R4-F | I | C | I | P | F | H | T | | D |
| PR28 (SEQ ID NO: 30) | R5-F | I | C | I | P | F | H | T | | D |

| **Modified F4 and F9** | | | | | | | | | | |
|---|---|---|---|---|---|---|---|---|---|---|
| F4bhPhe (SEQ ID NO: 31) | D | T | H | bhPhe | P | I | C | I | | F |
| F4Dpa (SEQ ID NO: 32) | D | T | H | Dpa | P | I | C | I | | F |
| F4Bip | D | T | H | Bip | P | I | C | I | | F |
| (SEQ ID NO: 33) | | | | | | | | | | |
| F4 1Nal (SEQ ID NO: 34) | D | T | H | 1Nal | P | I | C | I | | F |
| F4bhDpa (SEQ ID NO: 35) | D | T | H | bhDpa | P | I | C | I | | F |
| F9bhPhe (SEQ ID NO: 36) | D | T | H | F | P | I | C | I | | bhPhe |
| F9Dpa (SEQ ID NO: 37) | D | T | H | F | P | I | C | I | | Dpa |
| F9Bip (SEQ ID NO: 38) | D | T | H | F | P | I | C | I | | Bip |
| F91Nal (SEQ ID NO: 39) | D | T | H | F | P | I | C | I | | 1Nal |
| F9bhDpa (SEQ ID NO: 40) | D | T | H | F | P | I | C | I | | bhDpa |
| PR39 (SEQ ID NO: 41) | D | T | H | Dpa | P | I | C | I | | Dpa |
| PR40 (SEQ ID NO: 42) | D | - | Dpa | - | P | I | C | I | | F |
| PR41 (SEQ ID NO: 43) | D | - | Dpa | - | P | I | C | I | | Dpa |
| PR43 (SEQ ID NO: 44) | D | T | H | Dpa | P | R | C | R | | Dpa |
| PR44 (SEQ ID NO: 45) | D | T | H | Dpa | Oic | I | C | I | | F |
| PR45 (SEQ ID NO: 46) | D | T | H | Dpa | Oic | I | C | I | | Dpa |
| PR46 (SEQ ID NO: 47) | D | T | H | Dpa | P | C | C | C | | Dpa |

| **Positive Charge** | | | | | | | | | | |
|---|---|---|---|---|---|---|---|---|---|---|
| PR13 (SEQ ID NO: 48) | D | T | H | F | P | I | C | I | | F-R8 |
| PR14 (SEQ ID NO: 49) | D | T | H | F | P | I | C | I | | F-R9 |
| PR15 (SEQ ID NO: 50) | D | T | H | F | P | I | C | I | | F-R10 |
| PR16 (SEQ ID NO: 51) | D | T | H | F | P | I | C | I | | F-R11 |
| PR17 | D | T | H | F | P | I | C | I | | F-R12 |
| (SEQ ID NO: 52) | | | | | | | | | | |
| PR18 (SEQ ID NO: 53) | D | T | H | F | P | I | C | I | F-R13 | - |
| PR19 (SEQ ID NO: 54) | D | T | H | F | P | I | C | I | bhPhe-R8 | - |
| PR20 (SEQ ID NO: 55) | D | T | H | F | P | I | C | I | bhPhe-R9 | - |
| PR21 (SEQ ID NO: 56) | D | T | H | F | P | I | C | I | bhPhe-R12 | - |
| PR22 (SEQ ID NO: 57) | D | T | H | F | P | I | C | I | bhPhe-R13 | - |
| PR-1 (SEQ ID NO: 58) | C | Inp | (D)Dpa | Amc | R | Amc | Inp | Dpa | Cysteamide** | - |
| PR-2 (SEQ ID NO: 59) | C | P | (D)Dpa | Amc | R | Amc | Inp | Dpa | Cysteamide** | - |
| PR-3 (SEQ ID NO: 60) | C | P | (D)Dpa | Amc | R | Amc | Inp | Dpa | Cysteamide** | - |
| PR-4 (SEQ ID NO: 61) | C | G | (D)Dpa | Amc | R | Amc | Inp | Dpa | Cysteamide** | - |
| R1 = -CONH₂-CH₂-CH₂-S | | | | | | | | | | |
| R2 = Chenodeoxycholate-(D)Asp-(PEG11)- | | | | | | | | | | |
| R3 = Ursodeoxycholate-(D)Asp-(PEG11)- | | | | | | | | | | |
| R4 = Palmitoyl-(PEG11)- | | | | | | | | | | |
| R5 = (Palmitoyl)₂-Dap-PEG11- , wherein "Dap" = diaminopropionic acid | | | | | | | | | | |
| R6 = -(PEG11)-GYIPEAPRDGQAYVRKDGEWVLLSTFL (SEQ ID NO: 62) | | | | | | | | | | |
| R7 = -(PEG11)-(GPHyp)10 , "GPHyp" = Gly-Pro-hydroxyproline | | | | | | | | | | |
| R8 = -PPK | | | | | | | | | | |
| R9 = -PPR | | | | | | | | | | |
| R10 = -bhProPK | | | | | | | | | | |
| R11 = -bhProPR | | | | | | | | | | |
| R12 = -PbhProK | | | | | | | | | | |
| R13 = -PbhProR | | | | | | | | | | |
| Underlined residues = D amino acids | | | | | | | | | | |
| "-" indicates a covalent bond, e.g. point of attachment to the given peptide | | | | | | | | | | |
| ** oxidized | | | | | | | | | | |
| The PEG compound may be PEG11, i.e. O-(2-aminoethyl)-O'-(2-carboxyethyl)-undecaethyleneglycol PR12, riHep7ΔDT, PR23, PR24, PR25, PR26, PR27 and PR28 are retroinverted mini-hepcidins and are shown, left to right, from their C-terminus to their N-terminus. | | | | | | | | | | |

| **Table 3** | | | | | | | | | | |
|---|---|---|---|---|---|---|---|---|---|---|
| **Name** | **1** | **2** | **3** | **4** | **5** | **6** | **7** | **8** | **9** | **10** |
| Hep10wt (SEQ ID NO: 2) | D | T | H | F | P | I | C | I | F | C |
| PR42' (SEQ ID NO: 63) | D | T | H | Dpa | P | R | C | R | Dpa | |
| PR47 (SEQ ID NO: 64) | D | T | H | Dpa | P | I | C | I | F-R4 | |
| PR48 (SEQ ID NO: 65) | D | T | H | Dpa | P | I | C | I | Dpa-R4 | |
| PR49 (SEQ ID NO: 66) | | | H | Dpa | P | I | C | I | F-R4 | |
| PR50 (SEQ ID NO: 67) | | | H | Dpa | P | I | C | I | Dpa-R4 | |
| PR51 (SEQ ID NO: 68) | D | T | H | Dpa | P | V | C | V | F-R4 | |
| PR52 (SEQ ID NO: 69) | D | T | H | Dpa | P | L | C | L | F-R4 | |
| PR53 (SEQ ID NO: 70) | N-MeAsp | T | H | Dpa | P | I | C | I | bhPhe-R14 | |
| PR54 (SEQ ID NO: 71) | N-MeAsp | T | H | Dpa | bhPro | I | C | I | bhPhe-R14 | |
| PR55 (SEQ ID NO: 72) | N-MeAsp | T | H | Dpa | P | Ach | C | Ach | F-R14 | |
| PR56 (SEQ ID NO: 73) | N-MeAsp | T | H | Dpa | Oic | R | C | R | bhPhe-R14 | |
| PR57 (SEQ ID NO: 74) | N-MeAsp | T | H | Dpa | bhPro | R | C | R | bhPhe-R14 | |
| PR58 (SEQ ID NO: 75) | Ida | T | H | Dpa | P | I | C | I | bhPhe-R14 | |
| PR59 (SEQ ID NO: 76) | Ida | T | H | Dpa | bhPro | I | C | I | bhPhe-R14 | |
| PR60 (SEQ ID NO: 77) | Ida | T | H | Dpa | P | Ach | C | Ach | F-R14 | |
| PR61 (SEQ ID NO: 78) | Ida | T | H | Dpa | bhPro | R | C | R | bhPhe-R14 | |
| R4 = Palmitoyl-(PEG11)-, PEG1 = O-(2-aminoethyl)O'-(2-carboxyethyl)-undecaethyleneglycol | | | | | | | | | | |
| R14= Palmitoyl-PEG-miniPEG3-, and "miniPEG3" = 11-amino-3,6,9-trioxaundecanoic acid | | | | | | | | | | |
| Underlined residues = D amino acids | | | | | | | | | | |
| "-" indicates a covalent bond, e.g. point of attachment to the given peptide | | | | | | | | | | |
| In some embodiments, PEG11 can be substituted with miniPEG3 and miniPEG3 can be substituted with PEG11. | | | | | | | | | | |

| **Table 4** | | | | | | | | | | |
|---|---|---|---|---|---|---|---|---|---|---|
| **Name** | **1** | **2** | **3** | **4** | **5** | **6** | **7** | **8** | **9** | **10** |
| Hep10wt (SEQ ID NO: 2) | D | T | H | F | P | I | C | I | F | C |
| PR62 (SEQ ID NO: | Ida | T | H | Dpa | bhPro | R | C | R | bhPhe-R14 | |
| PR63 (SEQ ID NO: 80) | Ida | T | H | Dpa | bhPro | N-MeArg | C | N-MeArg | bhPhe-R14 | |
| PR64 (SEQ ID NO: 81) | Ida | T | H | Dpa | bhPro | bhArg | C | bhArg | bhPhe-R14 | |
| PR65 (SEQ ID NO: 82) | Ida | T | H | Dpa | bhPro | R | C | R | bhPhe-R15 | |
| PR66 (SEQ ID NO: 83) | Ida | T | H | Dpa | bhPro | R | C | R | bhPhe | |
| PR67 (SEQ ID NO: 84) | Ida | T | H | Dpa | bhPro | R | Cys(S-S-Pal) | R | bhPhe | |
| PR68 (SEQ ID NO: 85) | Ida | T | H | Dpa | bhPro | R | Cys(S-S-cysteamine-Pal) | R | bhPhe | |
| PR69 (SEQ ID NO: 86) | Ida | T | H | Dpa | bhPro | R | Cys(S-S-Cys-NHPal) | R | bhPhe | |
| PR70 (SEQ ID NO: 87) | Ida | T | H | Dpa | bhPro | R | Cys(S-S-Cys) | R | bhPhe-R14 | |
| PR71 (SEQ ID NO: 88) | Ida(NHPal) | T | H | Dpa | bhPro | R | C | R | bhPhe | |
| PR72 (SEQ ID NO: 89) | Ida | T | H | Dpa | bhPro | R | C | R | bhPhe | Ida(NHPal) |
| PR73 (SEQ ID NO: 90) | Ida | T | H | Dpa | bhPro | R | C | R | bhPhe | Ahx-Ida(NHPal) |
| PR74 (SEQ ID NO: 91) | Ida | T | H | Dpa | bhPro | R | C | R | bhPhe | Ahx-Ida(NBzl2) |
| PR75 (SEQ ID NO: 92) | Ida | T | H | Dpa | bhPro | R | C | R | bhPhe-R16 | |
| PR76 (SEQ ID NO: | D | T | H | F | P | R | Cys(S-S-tBut) | R | W-R17 | |
| PR77 (SEQ ID NO: | D | T | H | F | P | R | Cys(S-S-tBut) | R | W-R18 | |
| PR78 (SEQ ID NO: | D | T | H | F | P | R | Cys(S-S-tBut) | R | W-R19 | |
| PR79 (SEQ ID NO: | D | T | H | F | P | R | Cys(S-S-tBut) | R | W-R20 | |
| PR82 (SEQ ID NO: 97) | Ida | T | H | Dpa | bhPro | R | C | R | W | Ahx-Ida(NHPal) |
| PR83 (SEQ ID NO: 98) | D | T | H | F | P | R | C | R | D | |
| PR84 (SEQ ID NO: 99) | D | T | H | F | P | R | C | R | | |
| PR85 (SEQ ID NO: 100) | D | T | H | F | P | R | C | R | D | |
| PR86 (SEQ ID NO: 101) | D | T | H | F | P | R | C | R | | |
| R4 = Palmitoyl-(PEG11)-, wherein PEG11 = O-(2-aminoethyl)-O'-(2-carboxyethyl)-undecaethyleneglycol | | | | | | | | | | |
| R14 = Palmitoyl-PEG-miniPEG3-, and "miniPEG3" = 11-amino-3,6,9-trioxaundecanoic acid | | | | | | | | | | |
| R15 = Palmitoyl-PEG- | | | | | | | | | | |
| R16 = C16 | | | | | | | | | | |
| R17 = Butanoyl-PEG11- | | | | | | | | | | |
| R18 = Octanoyl-PEG11- | | | | | | | | | | |
| R19 = Palmitoyl-PEG11- | | | | | | | | | | |
| R20 = Tetracosanoyl-PEG11- | | | | | | | | | | |
| Ahx-Ida(NHPal) = Aminohexanoic acid spacer between peptide residue 9 and Ida residue; Palmitylamine amide on Ida side chain | | | | | | | | | | |
| Ida(NHPal) = Palmitylamine amide on Ida side chain | | | | | | | | | | |
| Ida(NBzl2) = N,N'-Dibenzylamine amide on Ida side chain | | | | | | | | | | |
| Cys(S-S-Pal) = Palmitoyl attached to Cys7 via a disufide bond | | | | | | | | | | |
| Cys(S-S-cysteamine-Pal) = Palmitoyl attached to Cys7 via SS-Cysteamine | | | | | | | | | | |
| Cys(S-S-Cys-NHPal) = Palmitylamine attached to Cys7 via another Cys | | | | | | | | | | |
| Cys(S-S-Cys) = Cys attached to Cys7 via disulfide bond | | | | | | | | | | |
| Underlined residues = D amino acids | | | | | | | | | | |
| "-" indicates a covalent bond, e.g. point of attachment to the given peptide | | | | | | | | | | |
| In some embodiments, PEG11 can be substituted with miniPEG3. | | | | | | | | | | |
| In some embodiments, miniPEG3 can be substituted with PEG11. | | | | | | | | | | |
| In some embodiments, PEG can be substituted with PEG11, but not miniPEG3. | | | | | | | | | | |

| **TABLE 5** | | | | | |
|---|---|---|---|---|---|
| **Uncommon or Unnatural Amino Acids** | | | | | |
| 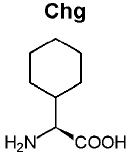 | 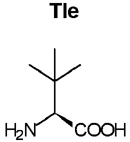 | 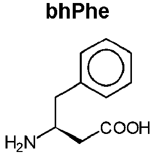 | 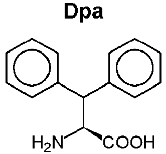 | 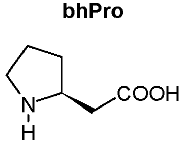 | 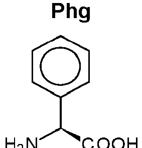 |
| L-α-cyclohexylglycine | L-tert-leucine | β-homophenylalanine | 3,3-diphenyl-L-alanine | L-beta-homoproline | L-phenylglycine |
| 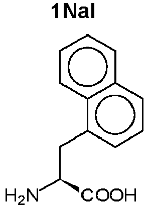 | 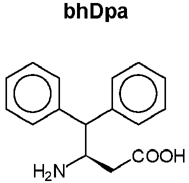 | 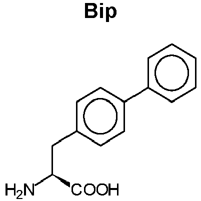 | 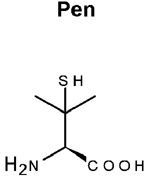 | 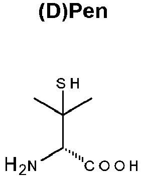 | 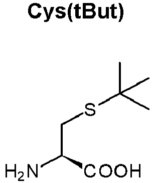 |
| (1-naphthyl)-L-alanine | (S)-3-Amino-4,4-diphenylbutanoic acid | L-biphenylalanine | L-Penicillamine | D-Penicillamine | S-t-butyl-L-cysteine |
| 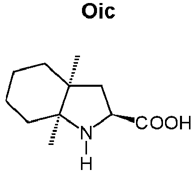 | 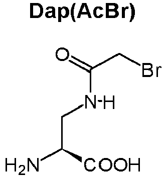 | 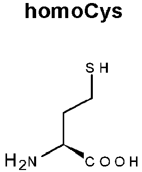 | 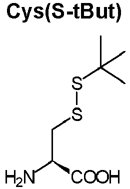 | 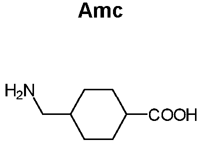 | 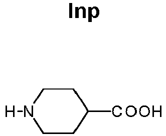 |
| octahydroindole-2-carboxylic acid | N^{γ}-(bromoacetyl)-L -2,3-diaminopropionic acid | L-homocysteine | S-t-Butylthio-L-cysteine | 4-(aminomethyl)cyclohex ane carboxylic acid | isonipecotic acid |
| **bhAsp** | **Ida** | **N-MeAsp** | **N-MeThr** | **2-Aminoindane** | **PheF5** |
| 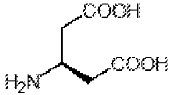 | 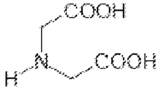 | 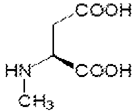 | 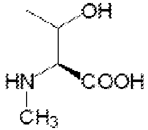 | 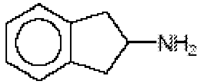 | 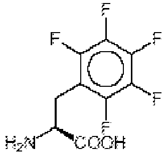 |
| **hPhe** | **Igl** | **trans-4-PhPro** | **cis-4-PhPro** | **cis-5-PhPro** | **Idc** |
| 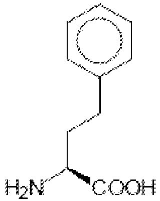 | 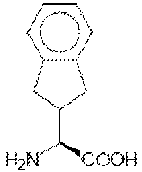 | 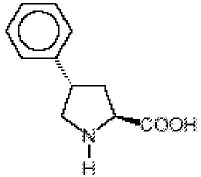 | 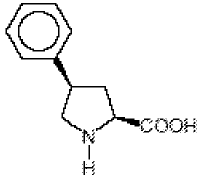 | 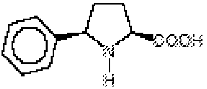 | 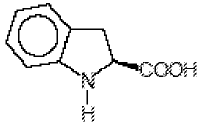 |
| **bhIle** | **Ach** | **N-Melle** | **N-MePhe** | **Benzulamide** | **(D)Dpa** |
| 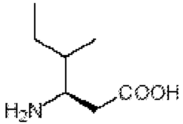 | 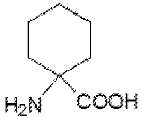 | 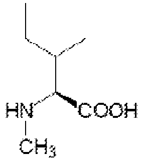 | 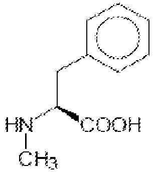 | 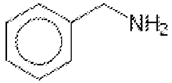 | 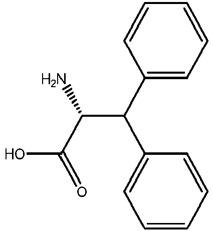 |
| | | | | | 3,3-diphenyl-D-alanine |
| **Ahx** | **N-MeArg** | | **2Nal** | **L-His(π-Me)** | **L-His(τ-Me)** |
| 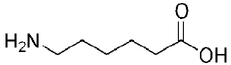 | 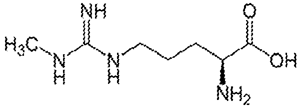 | | 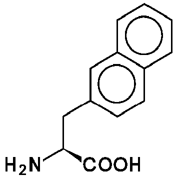 | 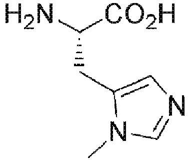 | 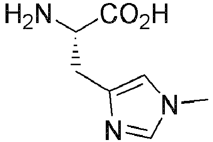 |

As provided herein, a bond is represented by a line, such as "-", or the symbol " 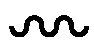 ". The line and symbol represent that the bond is the point of attachment between two molecular subunits. As used herein, usage of "(Cₙ-Cₘ)" indicates the range of carbon atoms the indicated hydrocarbon may have. For example, the term "(C₁-C₆)alkyl" refers to a straight or branched hydrocarbon from 1 to 6 carbon atoms and includes methyl, ethyl, n-propyl, isopropyl, n-butyl, sec-butyl, isobutyl, tert-butyl, n-pentyl, n-hexyl, and the like. Similarly, usage of "(Cₙ)" indicates the number of carbon atoms the indicated hydrocarbon contains.

An "alkyl" refers to a straight or branched chain monovalent radical of saturated and/or unsaturated carbon atoms and hydrogen atoms, such as methyl (Me) ethyl (Et) propyl (Pr) isopropyl (i-Pr) butyl (n-Bu) isobutyl (i-Bu) t-butyl (t-Bu) (sec-Bu) ethenyl, pentenyl, butenyl, propenyl, ethynyl, butynyl, propynyl, pentynyl, hexynyl, and the like, which may be unsubstituted (i.e., contain only carbon and hydrogen) or substituted by one or more substituents as defined below. The term "(C₁-C₆)alkyl" as used herein refers to a straight or branched hydrocarbon from 1 to 6 carbon atoms and includes methyl, ethyl, n-propyl, isopropyl, n-butyl, sec-butyl, isobutyl, tert-butyl, n-pentyl, n-hexyl, and the like. The (C₁-C₆)alkyl group optionally can be substituted with one or more substituents as defined below. The term "(C₁-C₃)alkyl" as used herein refers to a straight or branched hydrocarbon of from 1 to 3 carbon atoms and includes methyl, ethyl, n-propyl, isopropyl, and the like. The (C₁-C₃)alkyl group optionally can be substituted with one or more of more substituents as defined below.

An "alkoxy" refers to the radical -OR, where R is a straight or branched chain alkyl group. Exemplary alkoxy groups include methoxy, ethoxy, n-propoxy, isopropoxy, n-butoxy, isobutoxy, pentoxy, and the like. A "(C₁-C₆)alkoxy" refers to a straight or branched chain alkoxy group containing from 1 to 6 carbon atoms and a "(C₁-C₃)alkoxy" refers to a straight or branched chain alkoxy group containing from 1 to 3 carbon atoms.

An "alkoxycarbonyl" refers to the radical -C(O)OR, where Ris an alkyl group.

A "cycloalkyl" refers to a non-aromatic monovalent monocyclic, bicyclic, or tricyclic radical comprising 3-14 carbon ring atoms, each of which may be saturated or unsaturated, and which may be unsubstituted or substituted by one or more suitable substituents as defined below, and to which may be fused one or more heterocycloalkyl groups, aryl groups, or heteroaryl groups, which themselves may be unsubstituted or substituted by one or more substituents. The term "(C₃-C₈)cycloalkyl" means a hydrocarbon ring containing from 3 to 8 carbon atoms, for example, cyclopropyl, cyclobutyl, cyclopentyl, or cyclohexyl. Where possible, the cycloalkyl group may contain double bonds, for example, 3-cyclohexen-1-yl. The cycloalkyl ring may be unsubstituted or optionally may be substituted by one or more substituents selected from (C₁-C₆)alkyl, (C₁-C₆)alkoxy, (C₁-C₆)thioalkoxy, hydroxy, thiol, halo, formyl, carboxyl, amino, aminoalkyl, -CO₂(C₁-C₆)alkyl, -CO(C₁-C₆)alkyl, -C(O)N(C₁-C₆)alkyl, aryl, and heteroaryl.

An "aryl" refers to a cyclic or polycyclic aromatic ring having from 5 to 12 carbon atoms, and may be unsubstituted or substituted by one or more suitable substituents as defined below, and to which may be fused one or more cycloalkyl groups, heterocycloalkyl groups, or heteroaryl groups, which themselves may be unsubstituted or substituted by one or more suitable substituents.

A "heteroaryl" refers to an aromatic monovalent monocyclic, bicyclic, or tricyclic radical comprising 4-18 ring members, including 1-5 heteroatoms selected from nitrogen, oxygen, and sulfur, which may be unsubstituted or substituted by one or more suitable substituents as defined below, and to which may be fused one or more cycloalkyl groups, heterocycloalkyl groups, or aryl groups, which themselves may be unsubstituted or substituted by one or more suitable substituents.

A "heterocycloalkyl" refers to a non-aromatic monovalent monocyclic, bicyclic, or tricyclic radical, which is saturated or unsaturated, comprising 3-18 ring members, which includes 1-5 heteroatoms selected from nitrogen, oxygen, and sulfur, where the radical is unsubstituted or substituted by one or more suitable substituents as defined below, and to which may be fused one or more cycloalkyl groups, aryl groups, or heteroaryl groups, which themselves may be unsubstituted or substituted by one or more suitable substituents.

An "acyl" refers to a -C(O)-R radical, where R is a suitable substituent as defined below.

A "sulfonyl" refers to a -SO₂R radical, where R is a suitable substituent as defined below.

An "alkylsulfonyl" refers to the radical -SO₂R, where Ris an alkyl group.

An "alkylamino" refers to an amino moiety substituted with one (i.e., -NHR) or two (i.e., -NRR') (C₁-C₆)alkyl groups which may be the same or different. Examples of such alkylamino groups include aminomethyl, dimethylamino, aminomethylethyl, aminomethylpropyl, and the like.

An "alkylaminocarbonyl" refers to the radical -C(O)NHR, where Ris an alkyl group.

A "dialkylaminocarbonyl" refers to the radical -C(O)NRR', where each R may be the same or different alkyl group.

A "carboxyl" refers to the radical -C(O)OH.

A "carbamoyl group" refers to the radical C(O)NH₂.

In general, the various moieties or functional groups for variables in the formulae may be "optionally substituted" by one or more suitable "substituents". The term "substituent" or "suitable substituent" refers to any suitable substituent that may be recognized or selected, such as through routine testing, by those skilled in the art. In some embodiments, the substituent is N, O, Si, P, or S.

As used herein, a "disease of iron metabolism" includes diseases where aberrant iron metabolism directly causes the disease, or where iron blood levels are dysregulated causing disease, or where iron dysregulation is a consequence of another disease, or where diseases can be treated by modulating iron levels, and the like. More specifically, a disease of iron metabolism according to this disclosure includes iron overload diseases, iron deficiency disorders, disorders of iron biodistribution, other disorders of iron metabolism and other disorders potentially related to iron metabolism, etc. Diseases of iron metabolism include hemochromatosis, HFE mutation hemochromatosis, ferroportin mutation hemochromatosis, transferrin receptor 2 mutation hemochromatosis, hemojuvelin mutation hemochromatosis, hepcidin mutation hemochromatosis, juvenile hemochromatosis, neonatal hemochromatosis, hepcidin deficiency, transfusional iron overload, thalassemia, thalassemia intermedia, alpha thalassemia, sideroblastic anemia, polycythemia vera, myelodysplastic syndromes, porphyria, porphyria cutanea tarda, African iron overload, hyperferritinemia, ceruloplasmin deficiency, atransferrinemia, congenital dyserythropoietic anemia, anemia of chronic disease, anemia of inflammation, anemia of infection, hypochromic microcytic anemia, iron-deficiency anemia, iron-refractory iron deficiency anemia, anemia of chronic kidney disease, erythropoietin resistance, iron deficiency of obesity, other anemias, benign or malignant tumors that overproduce hepcidin or induce its overproduction, conditions with hepcidin excess, Friedreich ataxia, gracile syndrome, Hallervorden-Spatz disease, Wilson's disease, pulmonary hemosiderosis, hepatocellular carcinoma, cancer, hepatitis, cirrhosis of liver, pica, chronic renal failure, insulin resistance, diabetes, atherosclerosis, neurodegenerative disorders, multiple sclerosis, Parkinson's disease, Huntington's disease, and Alzheimer's disease. As used herein, "iron overload diseases" and "diseases of iron overload" refer diseases and disorders that result in or may cause abnormally high levels of iron in afflicted subjects if untreated.

In some cases the diseases and disorders included in the definition of "disease of iron metabolism" are not typically identified as being iron related. For example, hepcidin is highly expressed in the murine pancreas suggesting that diabetes (Type I or Type II), insulin resistance, glucose intolerance, and other disorders may be ameliorated by treating underlying iron metabolism disorders. See Ilyin, G. et al. (2003) FEBS Lett. 542 22-26. As such, these diseases are encompassed under the broad definition. Those skilled in the art are readily able to determine whether a given disease is a "disease or iron metabolism" according to the present invention using methods known in the art, including the assays of WO 2004092405 and assays which monitor hepcidin, hemojuvelin, or iron levels and expression, which are known in the art such as those described in U.S. Patent No. 7,534,764. In some embodiments of the present invention, the diseases of iron metabolism are iron overload diseases, which include hereditary hemochromatosis, iron-loading anemias, alcoholic liver diseases and chronic hepatitis C.

As used herein, a compound having "hepcidin activity" means that the compound has the ability to lower plasma iron concentrations in subjects (e.g. mice or humans), when administered thereto (e.g. parenterally injected or orally administered), in a dose-dependent and time-dependent manner. See e.g. as demonstrated in Rivera et al. (2005), Blood 106:2196-9.

In some embodiments, the peptides of the present invention have *in vitro* activity as assayed by the ability to cause the internalization and degradation of ferroportin in a ferroportin-expressing cell line as taught in Nemeth et al. (2006) Blood 107:328-33. *In vitro* activity may be measured by the dose-dependent loss of fluorescence of cells engineered to display ferroportin fused to green fluorescent protein as in Nemeth et al. (2006) Blood 107:328-33. Aliquots of cells are incubated for 24 hours with graded concentrations of a reference preparation of Hep25 or the S-alkylated hepcidin peptide to be tested. As provided herein, the EC₅₀ values are provided as the concentration of a given compound (e.g. peptide) that elicits 50% of the maximal loss of fluorescence generated by the reference Hep25 preparation. EC₅₀ of Hep25 preparations in this assay range from 5 to 15 nM and some preferred S-alkylated hepcidin peptides have EC₅₀ values in *in vitro* activity assays of about 1,000 nM or less.

Other methods known in the art for calculating the hepcidin activity and *in vitro* activity of peptides according to the present invention may be used. For example, the in *vitro* activity of compounds may be measured by their ability to internalize cellular ferroportin, which is determined by immunohistochemistry or flow cytometry using antibodies which recognizes extracellular epitopes of ferroportin. Alternatively, the *in vitro* activity of compounds may be measured by their dose-dependent ability to inhibit the efflux of iron from ferroportin-expressing cells that are preloaded with radioisotopes or stable isotopes of iron, as in Nemeth et al. (2006) Blood 107:328-33.

One or more S-alkylated hepcidin peptides according to the present invention, alone or in combination with one or more mini-hepcidins and/or one or more modified mini-hepcidins, may be administered to subjects in order to treat, e.g., inhibit and/or reduce, iron overload in subjects, such as humans. Therefore, S-alkylated hepcidin peptides according to the present invention may be used in medicaments and treatments in order to treat iron overload disorders, e.g. beta-thalassemia and hereditary hemochromatosis, by inhibiting and/or reducing iron overload in subjects. In some embodiments, at least one S-alkylated hepcidin peptide is administered to a subject before, during, after, or a combination thereof, symptoms of iron overload are observed and/or being diagnosed as having an iron overload disorder.

In some embodiments, one or more S-alkylated hepcidin peptides, alone or in combination with one or more mini-hepcidins and/or modified mini-hepcidins, are provided in the form of a composition which comprises a carrier suitable for its intended purpose. The compositions may also include one or more additional ingredients suitable for its intended purpose. For example, for assays, the compositions may comprise liposomes, niclosamide, SL220 solubilization agent (NOF, Japan), cremophor EL (Sigma), ethanol, and DMSO. For treatment of an iron overload disease, the compositions may comprise different absorption enhancers and protease inhibitors, solid microparticles or nanoparticles for peptide encapsulation (such as chitosan and hydrogels), macromolecular conjugation, lipidization and other chemical modification.

The present invention also provides kits comprising one or more S-alkylated hepcidin peptides, alone or in combination with one or more mini-hepcidins, one or more modified mini-hepcidins, and/or compositions of the present invention packaged together with reagents, devices, instructional material, or a combination thereof. For example, the kits may include reagents used for conducting assays, drugs, and compositions for diagnosing, treating, or monitoring disorders of iron metabolism, devices for obtaining samples to be assayed, devices for mixing reagents and conducting assays, and the like.

As the S-alkylated hepcidin peptides of the present invention exhibit hepcidin activity, i.e., act as agonists of ferroportin degradation, one or more S-alkylated hepcidin peptides, alone or in combination with one or more mini-hepcidins and/or modified mini-hepcidins, may be used to treat iron overload diseases. For example, one or more S-alkylated hepcidin peptides, alone or in combination with one or more mini-hepcidins and/or modified mini-hepcidins, may be administered to a subject to ameliorate the symptoms and/or pathology associated with iron overload in iron-loading anemias (especially β-thalassemias) where phlebotomy is contraindicated and iron chelators are the mainstay of treatment but are often poorly tolerated. One or more S-alkylated hepcidin peptides, alone or in combination with one or more mini-hepcidins and/or modified mini-hepcidins, may be used to treat hereditary hemochromatosis, especially in subjects who do not tolerate maintenance phlebotomy. One or more S-alkylated hepcidin peptides, alone or in combination with one or more mini-hepcidins and/or modified mini-hepcidins, may be used to treat acute iron toxicity. In some embodiments, treatment with one or more S-alkylated hepcidin peptides, alone or in combination with one or more mini-hepcidins and/or modified mini-hepcidins, may be combined with phlebotomy or chelation.

One or more S-alkylated hepcidin peptides, alone or in combination with one or more mini-hepcidins and/or modified mini-hepcidins, may be administered to a subject, preferably a mammal such as a human. In some embodiments, the administration to the subject is before, during, and/or after the subject exhibits an increase in iron levels and/or abnormally high levels of iron. In some embodiments, the subject to be treated is one who is at risk of having high levels of iron and/or has a genetic predisposition to having an iron overload disease. In some embodiments, the peptides are administered in a form of a pharmaceutical composition. In some embodiments, the peptides are administered in a therapeutically effective amount. As used herein, a "therapeutically effective amount" is an amount which ameliorates the symptoms and/or pathology of a given disease of iron metabolism as compared to a control such as a placebo.

A therapeutically effective amount may be readily determined by standard methods known in the art. The dosages to be administered can be determined by one of ordinary skill in the art depending on the clinical severity of the disease, the age and weight of the subject, or the exposure of the subject to iron. In some embodiments, therapeutically effective amounts of S-alkylated hepcidin peptides range from about 0.01 to about 10 mg/kg body weight, about 0.01 to about 3 mg/kg body weight, about 0.01 to about 2 mg/kg, about 0.01 to about 1 mg/kg, or about 0.01 to about 0.5 mg/kg body weight for parenteral formulations. In some embodiments, therapeutically effective amounts for oral administration may be up to about 10-fold higher. Moreover, treatment of a subject with a peptide or composition of the present invention can include a single treatment or, preferably, can include a series of treatments. It will be appreciated that the actual dosages will vary according to the particular peptide or composition, the particular formulation, the mode of administration, and the particular site, host, and disease being treated. It will also be appreciated that the effective dosage used for treatment may increase or decrease over the course of a particular treatment. Optimal dosages for a given set of conditions may be ascertained by those skilled in the art using conventional dosage-determination tests in view of the experimental data for a given peptide or composition. Changes in dosage may result and become apparent by standard diagnostic assays known in the art. In some conditions chronic administration may be required.

The pharmaceutical compositions of the invention may be prepared in a unit-dosage form appropriate for the desired mode of administration. The compositions of the present invention may be administered for therapy by any suitable route including oral, rectal, nasal, topical (including buccal and sublingual), vaginal and parenteral (including subcutaneous, intramuscular, intravenous and intradermal). A variety of administration routes can be used in accordance with the present invention, including oral, topical, transdermal, nasal, pulmonary, transpercutaneous (wherein the skin has been broken either by mechanical or energy means), rectal, buccal, vaginal, via an implanted reservoir, or parenteral. Parenteral includes subcutaneous, intravenous, intramuscular, intraperitoneal, intra-articular, intra-synovial, intrasternal, intrathecal, intrahepatic, intralesional, and intracranial injection or infusion techniques, as well as injectable materials (including polymers) for localized therapy. In some embodiments, the route of administration is subcutaneous. In some embodiments, the composition is in a sealed sterile glass vial. In some embodiments, the composition contains a preservative. Pharmaceutical compositions may be formulated as bulk powder, tablets, liquids, gels, lyophilized, and the like, and may be further processed for administration. See e.g., REMINGTON: THE SCIENCE AND PRACTICE OF PHARMACY. 20th ed. (2000) Lippincott Williams & Wilkins. Baltimore, MD, and subsequent editions.

It will be appreciated that the preferred route will vary with the condition and age of the recipient, the nature of the condition to be treated, and the chosen peptide and composition. Pharmaceutical compositions of the present invention comprise a therapeutically effective amount of at least one peptide as disclosed herein, and a pharmaceutically acceptable carrier or diluent, which may be inert. As used herein the language "pharmaceutically acceptable carrier" is intended to include any and all solvents, dispersion media, bulking agent, coatings, antibacterial and antifungal agents, preservatives, isotonic and absorption delaying agents, and the like, compatible with pharmaceutical administration and known in the art. Except insofar as any conventional media or agent is incompatible with the active compound, use thereof in the compositions is contemplated.

Supplementary compounds can also be incorporated into the compositions. Supplementary compounds include niclosamide, liposomes, SL220 solubilization agent (NOF, Japan), Cremophor EL (Sigma), ethanol, and DMSO.

Toxicity and therapeutic efficacy of the peptides and compositions of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Peptides which exhibit large therapeutic indices are preferred. While peptides that exhibit toxic side effects may be used, care should be taken to design a delivery system that targets such peptides to the site of affected tissue in order to minimize potential damage to uninfected cells and, thereby, reduce side effects.

The data obtained from the cell culture assays and animal studies can be used in formulating a range of dosage for use in humans. The dosage of peptides of the present invention lies preferably within a range of circulating concentrations that include the ED₅₀ with little or no toxicity. The dosage may vary within this range depending upon the dosage form employed and the route of administration utilized. For any peptide used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography or by mass spectroscopy.

The resulting decrease of plasma iron could also reduce the levels of toxic non-transferrin bound iron (NTBI) and promote the mobilization of iron from the heart and endocrine organs where iron excess is not tolerated. Thus, in some embodiments, one or more S-alkylated hepcidin peptides may be administered to a subject in order to reduce the levels of NTBI and/or promote the mobilization of iron from the heart and endocrine organs to other organs and tissues. In some embodiments, in established iron overload in human subjects, effective treatment with one or more S-alkylated hepcidin peptides may include more than one dose per day, a prolonged treatment period before a beneficial effect in liver iron can be detected, or may be combined with removal of iron by phlebotomy or chelation.

According to U.S. Food and Drug Administration dosing adjustment guidelines, the difference in metabolic rates between the mouse and human requires a conversion based on the Km factor which normalizes doses to body surface area (Reagan-Shaw S, et al. (2008) FASEB J 22(3):659-661). A human equivalent dose (HED) can be estimated by HED = animal dose (mg/kg) x (animal Km/human Km), where the Km for mouse and an adult human are 3 and 37, respectively. Thus, according to the present invention, a subcutaneous dose of an S-alkylated hepcidin peptide in a human could be up to about 50-100 µg/kg/d, about 75-125 µg/kg/d, or about 90-110 µg/kg/d, preferably about 100 µg/kg/d (as this dose is a readily administrable amount of peptide about three times the median basal dose of the most widely used peptide drug, subcutaneous insulin, commonly used at 0.75 U/kg/d or 33 µg/kg/d in type 2 diabetics (Rosenstock J, et al. (2001) Diabetes Care 24(4):631-636)). Of course, lower doses, as well as higher doses, depending on the particular mini-hepcidin, form of administration, formulation, the subject, and the degree of iron overload, may be administered to subject. In some embodiments, a therapeutically effective dose of one or more S-alkylated hepcidin peptides ranges from about 10-500 µg/kg/d. Again, lower doses, as well as higher doses, depending on the particular mini-hepcidin, form of administration, formulation, the subject, and the degree of iron overload, may be administered to subject.

As provided herein, S-alkylated hepcidin peptides according to the present invention may be used to inhibit, reduce, or treat iron overload in subjects at risk due to genetic defects or those who have already undergone iron depletion, but no longer tolerate chelation or venesection therapy. The S-alkylated hepcidin peptides according to the present invention may be used to treat a subject having β-thalassemia major and/or a subject having hepcidin levels that are higher than normal but are lower than what is appropriate for the degree of iron overload and the particular subject. For example, one or more S-alkylated hepcidin peptides according to the present invention may be used to treat a subject who suffers from hyperabsorption of dietary iron, but has normal levels of iron, in order to lower the amount of iron in the subject and offset the hyperabsorption. One or more S-alkylated hepcidin peptides according to the present invention may be used to treat ineffective erythropoiesis and improve anemia in subjects.

Because of the relatively small size of the S-alkylated hepcidin peptides of the present invention, the S-alkylated hepcidin peptides may be appropriately formulated and optimized for oral administration or administration by other noninvasive means such as those used for insulin administration (Roach P. (2008) Clinical Pharmacokinetics 47(9):595-610) such as inhalation, or transcutaneous delivery, or mucosal nasal or buccal delivery.

PR73SH appears to be remarkably stable in mildly oxidizing conditions as prolonged storage of the compound in DMSO (10 mM solution) at room temperature for 30 days shows very limited levels of decomposition or sulfide oxidation (99.5 ± 0.5% of stability, determined by LC/MS/MS experiments). Thus, the present invention also provides storage stable compositions comprising one or more S-alkylated hepcidin peptides.

Section headings are used for organizational purposes only and are not to be construed as defining or limiting the subject matter described. Unless explicitly provided otherwise, singular word forms include the plural forms. As used herein, the singular forms "a", "an", and "the" include plural referents unless the context clearly dictates otherwise. As used herein, "and/or" means "and" or "or". For example, "A and/or B" means "A, B, or both A and B" and "A, B, and/or C" means "A, B, C, or a combination thereof" and said "combination thereof" means "A and B, A and C, or B and C". As used herein, "or" can mean "and/or" unless stated otherwise or the context clearly dictates otherwise.

In the event of a discrepancy between the sequences set forth in the sequence listing and the Tables, the sequences in the Table are controlling.

Having thus described exemplary embodiments of the present invention, it should be noted by those skilled in the art that the within disclosures are exemplary only and that various other alternatives, adaptations, and modifications may be made within the scope of the present invention. Accordingly, the present invention is not limited to the specific embodiments as illustrated herein, but is only limited by the following claims.

## Claims

1. An S-alkylated hepcidin peptide comprising or consisting of the following Structural Formula IA or IB
A1-A2-A3-A4-A5-A6-A7-A8-A9-A10 IA
A10-A9-A8-A7-A6-A5-A4-A3-A2-A1 IB
wherein
A1 is Asp, D-Asp, Glu, D-Glu, pyroglutamate, D-pyroglutamate, Gln, D-Gln, Asn, D-Asn, bhAsp, Ida, Ida(NHPal), or N-MeAsp;
A2 is Thr, D-Thr, Ser, D-Ser, Val, D-Val, Ile, D-Ile, Ala, D-Ala, Tle, Inp, Chg, bhThr, or N-MeThr;
A3 is His, D-His, Asn, D-Asn, Arg, D-Arg, L-His(π-Me), D-His(π-Me), L-His(τ-Me), or D-His(τ-Me);
A4 is Phe, D-Phe, Leu, D-Leu, Ile, D-Ile, Trp, D-Trp, Tyr, D-Tyr, Phg, bhPhe, Dpa, Bip, INal, 2Nal, bhDpa, Amc, PheF5, hPhe, Igl, or cyclohexylalanine;
A5 is Pro, D-Pro, Ser, D-Ser, Oic, bhPro, trans-4-PhPro, cis-4-PhPro, cis-5-PhPro, or Idc,;
A6 is Arg, D-Arg, Ile, D-Ile, Leu, D-Leu, Thr, D-Thr, Lys, D-Lys, Val, D-Val, D-Nω,ω-dimethyl-arginine, L-Nω,ω-dimethyl-arginine, D-homoarginine, L-homoarginine, D-norarginine, L-norarginine, citrulline, a modified Arg wherein the guanidinium group is modified or substituted, Norleucine, norvaline, bhIle, Ach, N-MeArg, or N-MeIle;
A8 is Arg, D-Arg, Ile, D-Ile, Leu, D-Leu, Thr, D-Thr, Lys, D-Lys, Val, D-Val, D-Nω,ω-dimethyl-arginine, L-Nω,ω-dimethyl-arginine, D-homoarginine, L-homoarginine, D-norarginine, L-norarginine, citrulline, a modified Arg wherein the guanidinium group is modified or substituted, Norleucine, norvaline, bhIle, Ach, N-MeArg, or N-MeIle;
A9 is Phe, D-Phe, Leu, D-Leu, Ile, D-Ile, Tyr, D-Tyr, Trp, D-Trp, Phe-R^{a}, D-Phe-R^{a}, Dpa-R^{a}, D-Dpa-R^{a}, Trp-R^{a}, bhPhe-R^{a}, PheF5, N-MePhe, benzylamide, 2-aminoindane, bhPhe, Dpa, Bip, INal, 2Nal, bhDpa, or cyclohexylalanine, which may or may not have R^{a} linked thereto, wherein R^{a} is palmitoyl-PEG-, wherein PEG is PEG11 or miniPEG3, palmitoyl-PEG-PEG, wherein PEG is PEG11 or miniPEG3, butanoyl (C4)-PEG11-, octanoyl (C8, Caprylic)-PEG11-, palmitoyl (C16)-PEG11-, or tetracosanoyl (C24, Lignoceric)-PEG11-; and
A10 is Cys, D-Cys, Ser, D-Ser, Ala, D-Ala, Ida, Ida(NHPal)Ahx, or Ida(NBzl2)Ahx; and A7 has Structural Formula A: 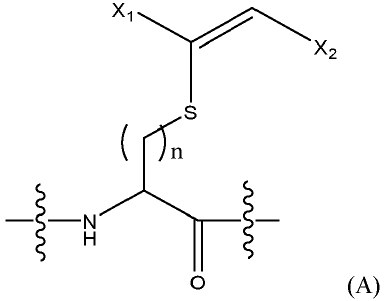 wherein
n is 1 or 2 and one or more of the hydrogens bonded to the Cn atom(s) may be substituted with a (C₁-C₃)alkyl,
X₁ and X₂ are each independently selected from the group consisting of H, alkyl, alkoxy, alkoxycarbonyl, cycloalkyl, aryl, heteroaryl, heterocycloalkyl, acyl, sulfonyl, alkylsulfonyl, alkylamino, alkylaminocarbonyl, dialkylaminocarbonyl, carboxyl, carbamoyl and 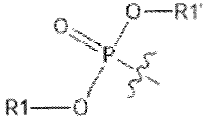 wherein R1 and R1' are each independently selected from the group consisting of H, methyl, (C₂)alkyl, (C₃)alkyl, (C₄)alkyl, (C₁-C₅)alkyl, (C₆)alkyl, (C₇)alkyl, (C₈)alkyl, (C₉)alkyl, and (C₁₀)alkyl;
wherein the carboxy-terminal amino acid is in amide or carboxy- form; and
wherein A1, A1 to A2, A10, or a combination thereof are optionally absent.

2. The S-alkylated hepcidin peptide according to claim 1, wherein the peptide comprises or consists of an amino acid sequence selected from SEQ ID NOs: 1-101 and having Structural Formula A as the A7 amino acid residue.

3. The S-alkylated hepcidin peptide according to claim 1, wherein the amino acid residue having Structural Formula A is A7, optionally wherein:
A1 is Ida, A2 is Thr, A3 is His, A4 is Dpa, A5 is bhPro, A6 is Arg, A8 is Arg, A9 is bhPhe, and A10 is Ahx-Ida(NHPal).

4. The S-alkylated hepcidin peptide according to claim 2, wherein the amino acid residue having Structural Formula A corresponds to a thiol containing amino acid of SEQ ID NOs: 1-101.

5. The S-alkylated hepcidin peptide according to any one of claims 1 to 4, wherein X₁ and X₂, are each independently selected from the group consisting of H, phenyl, 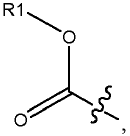 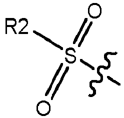 and 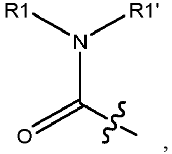 wherein R1 and R1' are each independently selected from the group consisting of H, methyl, (C₂)alkyl, (C₃)alkyl, (C₄)alkyl, (C₁-C₅)alkyl, (C₆)alkyl, (C₇)alkyl, (C₈)alkyl, (C₉)alkyl, and (C₁₀)alkyl; and R2 is -NR1R1', methyl, (C₂)alkyl, (C₃)alkyl, (C₄)alkyl, (C₁-C₅)alkyl, (C₆)alkyl, (C₇)alkyl, (C₈)alkyl, (C₉)alkyl, and (C₁₀)alkyl.

6. The S-alkylated hepcidin peptide according to claim 1 or claim 5, wherein R1 and R1' are each independently selected from the group consisting of H, methyl, ethyl, isopropyl, and tert-butyl.

7. The S-alkylated hepcidin peptide according to any one of claims 1 to 4, wherein X₁ and X₂ are each independently selected from the group consisting of H, phenyl, 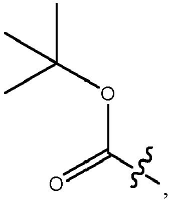 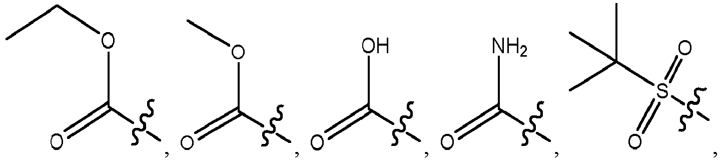 and

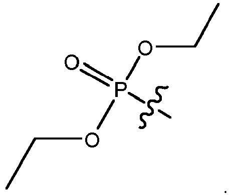

8. The S-alkylated hepcidin peptide according to any one of claims 1 to 4, wherein X₁ and X₂ are (a) both 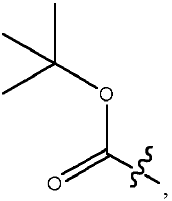 (b) both 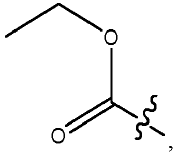 (c) both 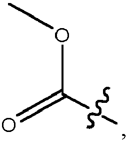 (c) H and 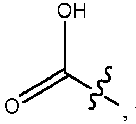 respectively, (d) phenyl and 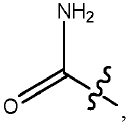 respectively, (e) both 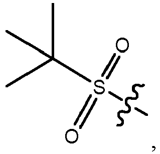 or (f) both

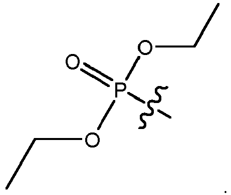

9. A composition which comprises at least one S-alkylated hepcidin peptide according to any one of claims 1 to 8.

10. An *in vitro* method of binding a ferroportin or inducing ferroportin internalization and degradation which comprises contacting the ferroportin with at least one S-alkylated hepcidin peptide according to any one of claims 1 to 8 or the composition according to claim 9.

11. The S-alkylated hepcidin peptide according to any one of claims 1 to 8, or the composition according to claim 9, for use in therapy.

12. A kit comprising at least one S-alkylated hepcidin peptide according to any one of claims 1 to 8 or the composition according to claim 9 packaged together with a reagent, a device, instructional material, or a combination thereof.

13. A complex comprising at least one S-alkylated hepcidin peptide according to any one of claims 1 to 8 bound to a ferroportin or an antibody.

14. The S-alkylated hepcidin peptide according to any one of claims 1 to 8, or the composition according to claim 9, for use in the treatment of a disease of iron metabolism, optionally iron overload disease, and/or lowering the amount of iron in a subject in need thereof.

15. The S-alkylated hepcidin peptides according to any one of claims 1 to 8, or the composition according to claim 9, for use as claimed in claim 14, wherein the peptide or composition is prepared to be administered at an effective daily dose as a single daily dose or as divided daily doses.

16. The S-alkylated hepcidin peptides according to any one of claims 1 to 8, or the composition according to claim 9, for use as claimed in claim 15, wherein the effective daily dose is about :-
(a) 10-500 µg/kg/day and the S-alkylated peptide or composition is formulated for subcutaneous injection: or
(b) 10-1000 µg/kg/day and the S-alkylated peptide or composition is formulated for oral, pulmonary, or mucosal administration.

## Patentansprüche

1. S-Alkyliertes Hepcidinpeptid, umfassend oder bestehend aus der folgenden Strukturformel IA oder IB
A1-A2-A3-A4-A5-A6-A7-A8-A9-A10 IA
A10-A9-A8-A7-A6-A5-A4-A3-A2-A1 IB
wobei
A1 Asp, D-Asp, Glu, D-Glu, Pyroglutamat, D-Pyroglutamat, Gln, D-Gln, Asn, D-Asn, bhAsp, Ida, Ida(NHPal) oder N-MeAsp ist;
A2 Thr, D-Thr, Ser, D-Ser, Val, D-Val, Ile, D-Ile, Ala, D-Ala, Tle, Inp, Chg, bhThr oder N-MeThr ist;
A3 His, D-His, Asn, D-Asn, Arg, D-Arg, L-His(π-Me), D-His(π-Me), L-His(τ-Me) oder D-His(τ-Me) ist;
A4 Phe, D-Phe, Leu, D-Leu, Ile, D-Ile, Trp, D-Trp, Tyr, D-Tyr, Phg, bhPhe, Dpa, Bip, 1Nal, 2Nal, bhDpa, Amc, PheF5, hPhe, Igl oder Cyclohexylalanin ist;
A5 Pro, D-Pro, Ser, D-Ser, Oic, bhPro, trans-4-PhPro, cis-4-PhPro, cis-5-PhPro oder Idc ist;
A6 Arg, D-Arg, Ile, D-Ile, Leu, D-Leu, Thr, D-Thr, Lys, D-Lys, Val, D-Val, D-Nω,ω-Dimethyl-Arginin, L-Nω,ω-Dimethyl-Arginin, D-Homoarginin, L-Homoarginin, D-Norarginin, L-Norarginin, Citrullin, ein modifiziertes Arg, wobei die Guanidinium-Gruppe modifiziert oder substituiert ist, Norleucin, Norvalin, bhIle, Ach, N-MeArg oder N-MeIle ist;
A8 Arg, D-Arg, Ile, D-Ile, Leu, D-Leu, Thr, D-Thr, Lys, D-Lys, Val, D-Val, D-Nω,ω-Dimethyl-Arginin, L-Nω,ω-Dimethyl-Arginin, D-Homoarginin, L-Homoarginin, D-Norarginin, L-Norarginin, Citrullin, ein modifiziertes Arg, wobei die Guanidinium-Gruppe modifiziert oder substituiert ist, Norleucin, Norvalin, bhIle, Ach, N-MeArg oder N-MeIle ist;
A9 Phe, D-Phe, Leu, D-Leu, Ile, D-Ile, Tyr, D-Tyr, Trp, D-Trp, Phe-R^{a}, D-Phe-R^{a}, Dpa-R^{a}, D-Dpa-R^{a}, Trp-R^{a}, bhPhe-R^{a}, PheF5, N-MePhe, Benzylamid, 2-Aminoindan, bhPhe, Dpa, Bip, 1Nal, 2Nal, bhDpa oder Cyclohexylalanin, an das R^{a} gebunden sein kann oder nicht, wobei R^{a} Palmitoyl-PEG- ist, wobei PEG PEG11 oder miniPEG3 ist, Palmitoyl-PEG-PEG, wobei PEG PEG11 oder miniPEG3 ist, Butanoyl (C4)-PEG11-, Octanoyl (C8, Caprylic)-PEG11Palmitoyl (C16)-PEG11- oder Tetracosanoyl (C24, Lignoceric)-PEG11- ist; und
A10 Cys, D-Cys, Ser, D-Ser, Ala, D-Ala, Ida, Ida(NHPal)Ahx oder Ida(NBzl2)Ahx ist; und
A7 die Strukturformel A aufweist: 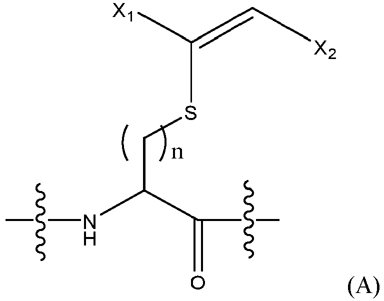 wobei
n 1 oder 2 ist und einer oder mehrere der an das (die) Cn-Atom(e) gebundenen Wasserstoffe mit einem (C₁-C₃)-Alkyl substituiert sein können,
X₁ und X₂ jeweils unabhängig ausgewählt sind aus der Gruppe bestehend aus H, Alkyl, Alkoxy, Alkoxycarbonyl, Cyclo-Alkyl, Aryl, Heteroaryl, Heterocyclo-Alkyl, Acyl, Sulfonyl, Alkylsulfonyl, Alkylamino, Alkylaminocarbonyl, Di-Alkylaminocarbonyl, Carboxyl,
Carbamoyl und 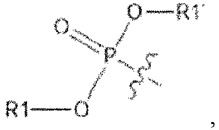 wobei R1 und R1' jeweils unabhängig ausgewählt sind aus der Gruppe, bestehend aus H, Methyl, (C₂)-Alkyl, (C₃)-Alkyl, (C₄)-Alkyl, (C₁-C₅)-Alkyl, (C₆)-Alkyl, (C₇)-Alkyl, (C₈)-Alkyl, (C₉)-Alkyl, und (C₁₀)-Alkyl;
wobei die carboxyterminale Aminosäure in Amid- oder Carboxyform ist; und
wobei A1, A1 bis A2, A10 oder eine Kombination davon optional abwesend sind.

2. S-Alkyliertes Hepcidinpeptid nach Anspruch 1, wobei das Peptid eine Aminosäuresequenz, ausgewählt aus SEQ ID NO: 1-101 umfasst oder daraus besteht und Strukturformel A als Aminosäurerest A7 aufweist.

3. S-Alkyliertes Hepcidinpeptid nach Anspruch 1, wobei der Aminosäurerest, der die Strukturformel A aufweist, A7 ist, optional wobei:
A1 Ida ist, A2 Thr ist, A3 His ist, A4 Dpa ist, A5 bhPro ist, A6 Arg ist, A8 Arg ist, A9 bhPhe ist und A10 Ahx-Ida(NHPal) ist.

4. S-Alkyliertes Hepcidinpeptid nach Anspruch 2, wobei der Aminosäurerest, der die Strukturformel A aufweist, einer thiolhaltigen Aminosäure von SEQ ID NO: 1-101 entspricht.

5. S-Alkyliertes Hepcidinpeptid nach einem der Ansprüche 1 bis 4, wobei X₁ und X₂ jeweils unabhängig ausgewählt sind aus der Gruppe, bestehend aus H, Phenyl, 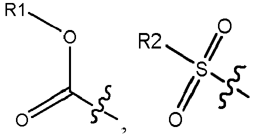 und 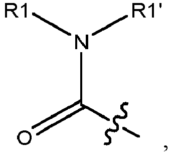 wobei R1 und R1' jeweils unabhängig ausgewählt sind aus der Gruppe, bestehend aus H, Methyl, (C₂)-Alkyl, (C₃)-Alkyl, (C₄)-Alkyl, (C₁-C₅)-Alkyl, (C₆)-Alkyl, (C₇)-Alkyl, (C₈)-Alkyl, (C₉)-Alkyl und (C₁₀)-Alkyl; und R2 -NR1R1', Methyl, (C₂)-Alkyl, (C₃)-Alkyl, (C₄)-Alkyl, (C₁-C₅)-Alkyl, (C₆)-Alkyl, (C₇)-Alkyl, (C₈)-Alkyl, (C₉)-Alkyl und (C₁₀)-Alkyl ist.

6. S-Alkyliertes Hepcidinpeptid nach Anspruch 1 oder Anspruch 5, wobei R1 und R1' jeweils unabhängig ausgewählt sind aus der Gruppe, bestehend aus H, Methyl, Ethyl, Isopropyl und tert-Butyl.

7. S-Alkyliertes Hepcidinpeptid nach einem der Ansprüche 1 bis 4, wobei X₁ und X₂ jeweils unabhängig ausgewählt sind aus der Gruppe, bestehend aus H, Phenyl, 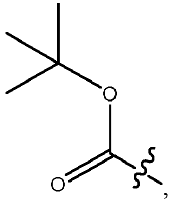 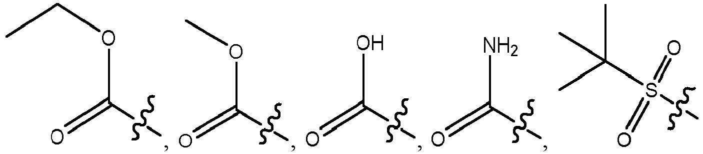 und

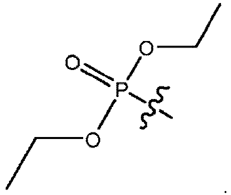

8. S-Alkyliertes Hepcidinpeptid nach einem der Ansprüche 1 bis 4, wobei X₁ und X₂ jeweils (a) beide 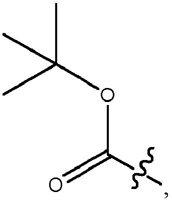 (b) beide 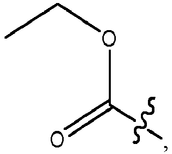 (c) beide 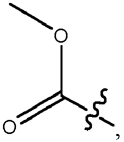 (c) H und 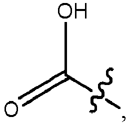 jeweils (d) Phenyl und 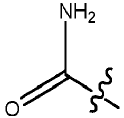 (e) beide 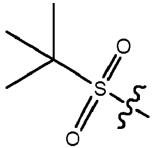 oder (f) beide 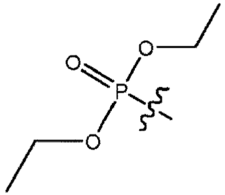 sind.

9. Zusammensetzung, die mindestens ein S-Alkyliertes Hepcidinpeptid nach einem der Ansprüche 1 bis 8 umfasst.

10. In-vitro-Verfahren zum Binden eines Ferroportins oder zum Induzieren einer Internalisierung und eines Abbaus von Ferroportin, das ein Inkontaktbringen des Ferroportins mit mindestens einem S-Alkylierten Hepcidinpeptid nach einem der Ansprüche 1 bis 8 oder der Zusammensetzung nach Anspruch 9 umfasst.

11. S-Alkyliertes Hepcidinpeptid nach einem der Ansprüche 1 bis 8 oder Zusammensetzung nach Anspruch 9 zur Verwendung in Therapie.

12. Kit, umfassend mindestens ein S-Alkyliertes Hepcidin-Peptid nach einem der Ansprüche 1 bis 8 oder die Zusammensetzung nach Anspruch 9, verpackt zusammen mit einem Reagenz, einer Vorrichtung, einem Anleitungsmaterial oder einer Kombination davon.

13. Komplex, umfassend mindestens ein S-Alkyliertes Hepcidin-Peptid nach einem der Ansprüche 1 bis 8, gebunden an ein Ferroportin oder einen Antikörper.

14. S-Alkyliertes Hepcidin-Peptid nach einem der Ansprüche 1 bis 8 oder Zusammensetzung nach Anspruch 9 zur Verwendung bei der Behandlung einer Erkrankung von Eisenstoffwechsel, optional einer Eisenüberlastungserkrankung, und/oder zum Senken der Eisenmenge bei einem Subjekt, das dies benötigt.

15. S-Alkylierte Hepcidinpeptide nach einem der Ansprüche 1 bis 8 oder Zusammensetzung nach Anspruch 9 zur Verwendung nach Anspruch 14, wobei das Peptid oder die Zusammensetzung zur Verabreichung in einer wirksamen Tagesdosis als einzelne Tagesdosis oder als geteilte Tagesdosen hergestellt ist.

16. S-Alkylierte Hepcidinpeptide nach einem der Ansprüche 1 bis 8 oder Zusammensetzung nach Anspruch 9 zur Verwendung nach Anspruch 15, wobei die wirksame Tagesdosis etwa wie folgt ist:
(a) 10-500 µg/kg/Tag und das S-Alkylierte Peptid oder die Zusammensetzung ist für eine subkutane Injektion formuliert: oder
(b) 10-1000 µg/kg/Tag und das S-Alkylierte Peptid oder die Zusammensetzung ist für eine orale, pulmonale oder mukosale Verabreichung formuliert.

## Revendications

1. Peptide d'hepcidine S-alkylé comprenant ou constitué par la Formule structurale IA ou IB suivante
A1-A2-A3-A4-A5-A6-A7-A8-A9-A10 IA
A10-A9-A8-A7-A6-A5-A4-A3-A2-A1 IB
dans lequel
A1 est Asp, D-Asp, Glu, D-Glu, un pyroglutamate, un D-pyroglutamate, Gln, D-Gln, Asn, D-Asn, bhAsp, Ida, Ida(NHPal), ou N-MeAsp ;
A2 est Thr, D-Thr, Ser, D-Ser, Val, D-Val, Ile, D-Ile, Ala, D-Ala, Tle, Inp, Chg, bhThr, ou N-MeThr ;
A3 est His, D-His, Asn, D-Asn, Arg, D-Arg, L-His(π-Me), D-His(π-Me), L-His(τ-Me), ou D-His(τ-Me) ;
A4 est Phe, D-Phe, Leu, D-Leu, Ile, D-Ile, Trp, D-Trp, Tyr, D-Tyr, Phg, bhPhe, Dpa, Bip, 1Nal, 2Nal, bhDpa, Amc, PheF5, hPhe, Igl, ou une cyclohexylalanine ;
A5 est Pro, D-Pro, Ser, D-Ser, Oic, bhPro, trans-4-PhPro, cis-4-PhPro, cis-5-PhPro, ou Idc,;
A6 est Arg, D-Arg, Ile, D-Ile, Leu, D-Leu, Thr, D-Thr, Lys, D-Lys, Val, D-Val, une D-Nω,ω-diméthyl-arginine, une L-Nω,ω-diméthyl-arginine, une D-homoarginine, une L-homoarginine, une D-norarginine, une L-norarginine, une citrulline, un Arg modifié dans lequel le groupe guanidinium est modifié ou substitué, une Norleucine, une norvaline, bhIle, Ach, N-MeArg, ou N-MeIle ;
A8 est Arg, D-Arg, Ile, D-Ile, Leu, D-Leu, Thr, D-Thr, Lys, D-Lys, Val, D-Val, une D-Nω,ω-diméthyl-arginine, une L-Nω,ω-diméthyl-arginine, une D-homoarginine, une L-homoarginine, une D-norarginine, une L-norarginine, une citrulline, un Arg modifié dans lequel le groupe guanidinium est modifié ou substitué, une Norleucine, une norvaline, bhIle, Ach, N-MeArg, ou N-Melle ;
A9 est Phe, D-Phe, Leu, D-Leu, Ile, D-Ile, Tyr, D-Tyr, Trp, D-Trp, Phe-R^{a}, D-Phe-R^{a}, Dpa-R^{a}, D-Dpa-R^{a}, Trp-R^{a}, bhPhe-R^{a}, PheF5, N-MePhe, un benzylamide, un 2-aminoindane, bhPhe, Dpa, Bip, 1Nal, 2Nal, bhDpa, ou une cyclohexylalanine, qui peut avoir ou ne pas avoir de R^{a} qui y est lié, dans lequel R^{a} est un palmitoyl-PEG-, dans lequel le PEG est un PEG11 ou un mini-PEG3, un palmitoyl-PEG-PEG, dans lequel le PEG est un PEG11 ou un mini-PEG3, un butanoyl (C4)-PEG11-, un octanoyl (C8, Caprylique)-PEG11-, un palmitoyl (C16)-PEG11-, ou un tétracosanoyl (C24, Lignocérique)-PEG11- ; et
A10 est Cys, D-Cys, Ser, D-Ser, Ala, D-Ala, Ida, Ida(NHPal)Ahx, ou Ida(NBzl2)Ahx ; et A7 a une Formule structurale A : 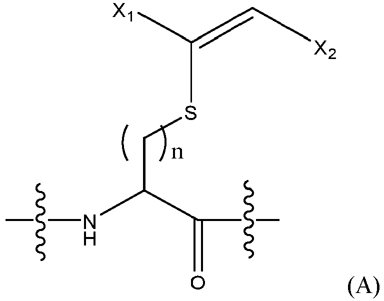 dans lequel
n vaut 1 ou 2 et un ou plusieurs des hydrogènes liés à ou aux atomes de Cn peuvent être substitués par un alkyle en (C₁ à C₃),
X₁ et X₂ sont chacun choisis indépendamment parmi le groupe constitué par H, un alkyle, un alcoxy, un alcoxycarbonyle, un cycloalkyle, un aryle, un hétéroaryle, un hétérocycloalkyle, un acyle, un sulfonyle, un alkylsulfonyle, un alkylamino, un alkylaminocarbonyle, un dialkylaminocarbonyle, un carboxyle, un carbamoyle et 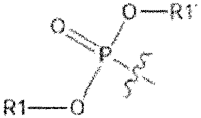 dans lequel R1 et R1' sont chacun choisis indépendamment parmi le groupe constitué par H, un méthyle, un alkyle en (C₂), un alkyle en (C₃), un alkyle en (C₄), un alkyle en (C₁ à C₅), un alkyle en (C₆), un alkyle en (C₇), un alkyle en (C₈), un alkyle en (C₉), et un alkyle en (C₁₀) ;
dans lequel l'acide aminé carboxy-terminal est sous une forme d'amide ou de carboxy ; et
dans lequel A1, A1 à A2, A10, ou une combinaison de ceux-ci sont facultativement absents.

2. Peptide d'hepcidine S-alkylé selon la revendication 1, dans lequel le peptide comprend ou est constitué par une séquence d'acides aminés choisie parmi les SEQ ID NO : 1 à 101 et ayant la Formule structurelle A comme le résidu d'acide aminé A7.

3. Peptide d'hepcidine S-alkylé selon la revendication 1, dans lequel le résidu d'acide aminé ayant la Formule structurale A est A7, facultativement dans lequel :
A1 est Ida, A2 est Thr, A3 est His, A4 est Dpa, A5 est bhPro, A6 est Arg, A8 est Arg, A9 est bhPhe, et A10 est Ahx-Ida(NHPal).

4. Peptide d'hepcidine S-alkylé selon la revendication 2, dans lequel le résidu d'acide aminé ayant la Formule structurale A correspond à un thiol contenant un acide aminé des SEQ ID NO : 1 à 101.

5. Peptide d'hepcidine S-alkylé selon l'une quelconque des revendications 1 à 4, dans lequel X₁ et X₂, sont chacun choisis indépendamment parmi le groupe constitué par H, un phényle, 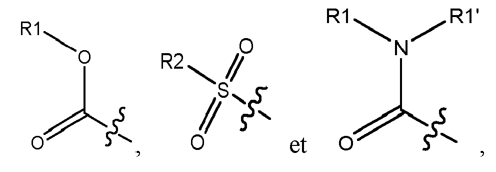 dans lequel R1 et R1' sont chacun choisis indépendamment parmi le groupe constitué par H, un méthyle, un alkyle en (C₂), un alkyle en (C₃), un alkyle en (C₄), un alkyle en (C₁ à C₅), un alkyle en (C₆), un alkyle en (C₇), un alkyle en (C₈), un alkyle en (C₉), et un alkyle en (C₁₀) ; et R2 est -NR1R1', un méthyle, un alkyle en (C₂), un alkyle en (C₃), un alkyle en (C₄), un alkyle en (C₁ à C₅), un alkyle en (C₆), un alkyle en (C₇), un alkyle en (C₈), un alkyle en (C₉), et un alkyle en (C₁₀).

6. Peptide d'hepcidine S-alkylé selon la revendication 1 ou la revendication 5, dans lequel R1 et R1' sont chacun choisis indépendamment parmi le groupe constitué par H, un méthyle, un éthyle, un isopropyle, et un tert-butyle.

7. Peptide d'hepcidine S-alkylé selon l'une quelconque des revendications 1 à 4, dans lequel X₁ et X₂ sont chacun choisis indépendamment parmi le groupe constitué par H, un phényle ,

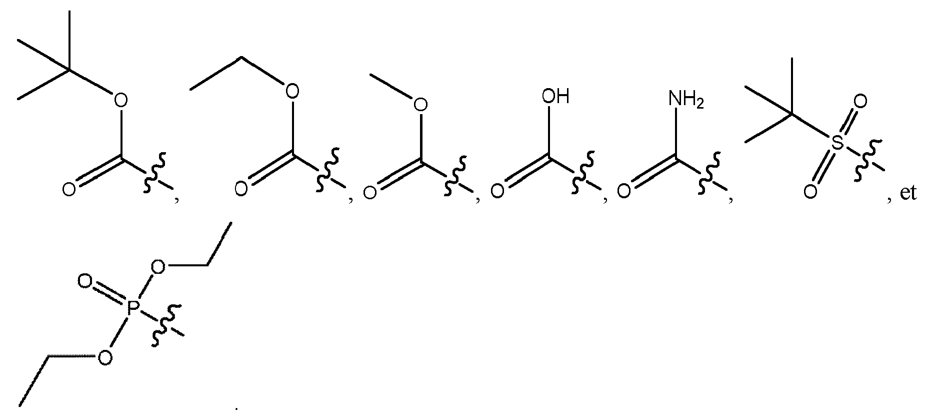

8. Peptide d'hepcidine S-alkylé selon l'une quelconque des revendications 1 à 4, dans lequel X₁ et X₂ sont (a) tous les deux 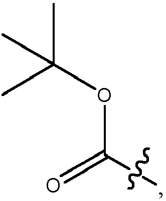 (b) tous les deux 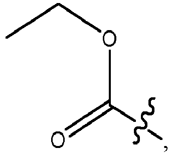 (c) tous les deux 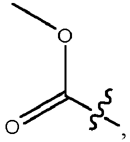 (c) H et 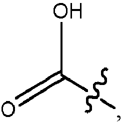 respectivement, (d) un phényle et 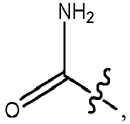 respectivement, (e) tous les deux 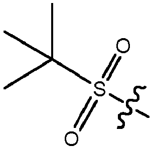 ou (f) tous les deux

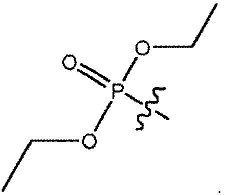

9. Composition qui comprend au moins un peptide d'hepcidine S-alkylé selon l'une quelconque des revendications 1 à 8.

10. Procédé *in vitro* de liaison d'une ferroportine ou d'induction de l'internalisation et de la dégradation de la ferroportine qui comprend la mise en contact de la ferroportine avec au moins un peptide d'hepcidine S-alkylé selon l'une quelconque des revendications 1 à 8 ou la composition selon la revendication 9.

11. Peptide d'hepcidine S-alkylé selon l'une quelconque des revendications 1 à 8, ou la composition selon la revendication 9, destiné à être utilisé en thérapie.

12. Trousse comprenant au moins un peptide d'hepcidine S-alkylé selon l'une quelconque des revendications 1 à 8 ou la composition selon la revendication 9 conditionné ensemble avec un réactif, un dispositif, un matériel d'instruction, ou une combinaison de ceux-ci.

13. Complexe comprenant au moins un peptide d'hepcidine S-alkylé selon l'une quelconque des revendications 1 à 8 lié à une ferroportine ou un anticorps.

14. Peptide d'hepcidine S-alkylé selon l'une quelconque des revendications 1 à 8, ou la composition selon la revendication 9, destiné à être utilisé dans le traitement d'une maladie du métabolisme du fer, facultativement une maladie de surcharge en fer, et/ou la réduction de la quantité de fer chez un sujet qui en a besoin.

15. Peptides d'hepcidine S-alkylés selon l'une quelconque des revendications 1 à 8, ou la composition selon la revendication 9, destinés à être utilisés selon la revendication 14, dans lesquels le peptide ou la composition est préparé pour être administré à une dose quotidienne efficace comme une dose quotidienne unique ou comme des doses quotidiennes divisées.

16. Peptides d'hepcidine S-alkylés selon l'une quelconque des revendications 1 à 8, ou la composition selon la revendication 9, destinés à être utilisés selon la revendication 15, dans lesquels la dose quotidienne efficace est d'environ :
(a) 10 à 500 µg/kg/jour et le peptide S-alkylé ou la composition est formulé pour une injection sous-cutanée ; ou
(b) 10 à 1 000 µg/kgjour et le peptide S-alkylé ou la composition est formulé pour une administration par voie orale, pulmonaire, ou muqueuse.
